# EUROPEAN PATENT APPLICATION

(11) **EP 4 793 959 A1**
(43) Date of publication of application: **19.08.2026**
(21) Application number: 26478001.6
(22) Date of filing: 17.02.2026
(51) Int. Cl.: G16H 10/65, G16H 10/60, G16H 80/00, G16H 30/20

(54) **DATA MANAGEMENT OF PERSONAL SERVER DEVICE**

(30) Priority: 14.02.2025 DK PA202530097
(71) Applicant: T&W miLINQ Aps, 3540 Lynge (DK)
(72) Inventor: ANDERSEN, Svend Vitting, Espergaerde, 3060 (DK); NIELSEN, Kim Hjortgaard, Frederikssund, 3660 (DK); WESTERMANN, Soeren Erik, Espergaerde, 3060 (DK); CHRISTENSEN, Thomas Evers, Holte, 2840 (DK)
(74) Representative: Wolgast, Carl Georg Love

(57) **Abstract**

A method (700) for managing limited temporary data storage in a portable device is provided.

The method (700) comprises steps of: continually receiving (S710) measurement data from at least one sensor (12), the measurement data comprising a time stamp corresponding to a time of measurement; assigning (S720) a first priority to the measurement data proportional to and increasing with an age of the time stamp; assigning (S730) a second priority to the measurement data proportional to and decreasing with the age of the time stamp; storing (S750) the measurement data in a primary memory buffer; transmitting (S760) measurement data for long-term storage in order according to the second priority; after receiving confirmation that the measurement data have been successfully transmitted, deleting (S770) the transmitted data; and when storage capacity in the primary memory buffer reaches a predetermined storage limit, deleting (S780) non-transmitted data in order according to the first priority.

A system (60) for managing limited temporary data storage in a portable personal server device (PS) (30) and a PS (30) for facilitating a secured and verified peer-to-peer bidirectional connection between one or more patient-specific medical devices (10) with at least one sensor (12) and a health care management system (20) are further provided.

## Description

### Technical Field

The present inventive concept relates to bidirectional communication between a patient-specific medical device with at least one sensor and a health care management system. In particular, the present inventive concept relates to methods, systems and devices for data management of secure, robust and verified real-time, long-distance communication of sensitive data.

### Background

Body-worn (battery operated) sensor devices are small, electronic devices that measure physiological and/or biokinetic data of human bodies. The data may either be stored for later analysis, transmitted to a remote storage/analysis system, or analysed immediately by the sensor device in order to generate some reaction/notification.

The latter two solutions require Remote Patient Monitoring (RPM) of a patient (typically having some form of physiological ailment). But RPM requires considerable processing power as well as radio communication technology (e.g. Bluetooth, Wi-Fi, ZigBee, IP, and 5G) and both of those need quite a lot of electrical power that is difficult to provide in batteries for (continuous) portable use.

At the same time, the data transport should be secure (verified sender/receiver identities) and protected from unintended use, which requires encryption of data and precautions against cyber-crime.

The communication should also be robust, i.e. as little data as possible should be lost between the sensor device and the final destination such as a health care management system.

Finally, it is beneficial if the (protected) data is communicated in standardized, unambiguous, and accepted data formats.

### Summary

It is thereby an object of the present inventive concept to provide a 'plug-in' wireless infrastructure that establishes a secure, real-time data link between one or more body-worn medical sensor devices and a healthcare management system (HCMS). The solution utilizes a 'digital twin' with internationally standardized data formats (e.g. DICOM) and an ultra-compact and effective peer-to-peer communication technology with IPv4 or IPv6 protocol compatibility and the system utilizes one or more data hubs to transfer the data via the internet. The technology allows for ultra-low latency transfer of data almost everywhere and at very low power consumption.

Should battery power run low, or communication broadband be temporarily reduced, the physiological and/or biokinetic data collected may need to be temporarily stored locally before it may be stored at an HCMS. By clever design, such a temporary storage may be especially efficient while taking into account the sensitive nature of the data being stored.

This and other objects are achieved by the features set out in the appended independent claims, with embodiments set out in the dependent claims.

Accordingly, a first aspect of the inventive concept is provided by a method for bidirectional communication between a patient-specific medical device with at least one sensor and a remote health care management system.

The method comprises: receiving measurement data from said at least one sensor; storing the measurement data (as current data) in a memory buffer; encrypting data in the memory buffer into encrypted data; establishing a secured and verified peer-to-peer connection between the patient-specific medical device and the remote health care management system; transmitting the encrypted data to the remote health care management system via the secured and verified peer-to-peer connection; decrypting the encrypted data (into decrypted, historical data); and updating a digital twin in the remote health care management system (in real-time) using the historical/decrypted data, the digital twin corresponding to (data of) a user of the medical device and comprising (a database or log of new and old) historical/decrypted data of said medical device.

The patient-specific medical device (MD) may be body-worn or not. The MD may be implanted or not. The MD may be portable or not.

If the MD is implanted, it may be difficult to communicate with the implanted MD through the skin of the patient using e.g. Bluetooth. There are safety regulations that may make it impossible to communicate directly with an implanted MD for risk of damaging human tissue.

For this reason, the MD may further comprise an external radio for communicating with the health care management system or a gateway device. The external radio may be attached to skin of the patient and in communication with the implant using e.g. inductance.

The health care management system (HCMS) may be a server or set of servers. The HCMS comprises the digital twin, security means, communication means, and possibly other items such as administrative means, a user interface, and relevant databases. The HCMS may be managed by the manufacturer of the MD, a service provider, an insurance provider, and/or by a hospital or health care provider. Third parties, such as a university, a relative, or a hospital or health care provider, may be allowed to interface with the HCMS to perform analysis on and/or receive updates about the medical conditions of the user. The data being accessible to third parties may be controlled by the HCMS, the data itself, the user, a medical professional, or some other authorized actor.

The term "current data" is data as they are received and buffered by the MD, which has not yet been transmitted to and verified by the HCMS.

The term "historical data" is data received and verified by the HCMS and are stored in the digital twin. The historical data may be as old as the latency of the transmission, which may be considered relatively low. Historical data may therefore be considered to be real-time data, as the historical data are as close to immediate as possible.

The latency of transmission may be optimized to be as low as possible while still possibly waiting for the memory buffer to reach a specific size optimal for packaging, encrypting and/or transmitting.

Historical data may be stored in the digital twin for as long as they are relevant. The digital twin may comprise a log or database of historical data. Historical data received via the secured and verified peer-to-peer connection may be assumed to be correct and authentic. Historical data are also decrypted data, as all medical data received by the HCMS are encrypted.

The digital twin may be set up in the HCMS as the user first activates or receives the MD. The digital twin may be associated with a specific user and related MD(s) as the digital twin is set up. The digital twin may be set up by a professional in a controlled environment, such as at a hospital or health care facility.

The digital twin may be set up in advance during manufacturing and later activated at home via an online interface by the user as a part of activating the MD for the first time. Activating the digital twin may comprise assigning an already set up digital twin to a user and/or an MD.

The digital twin may be (required to be) set up (again) after an update to the MD or its related or associated software.

Said at least one sensor may e.g. be an EEG sensor, ECG sensor, blood oximeter, glucose monitor, hearing aid, body microphone, temperature sensor, PPG sensor, accelerometer, hormone detector, chemical sensor, or any other suitable sensor of physiological conditions.

Said at least one sensor may produce measurement data such as EEG, ECG, blood pressure, soundscape, audiological environment, pulse, blood oxygenation, blood sugar, and/or any other measurable physiological attribute.

Storing the measurement data (as current data) may be done in a separate or integrated memory. The memory may be a part of the MD or a part of a separate device.

Encrypting the data may be done according to any number of ways known in the field of cryptography.

Establishing a secured and verified peer-to-peer connection may be done by a device remote to the HCMS such as a medical device, a personal server device or a connectivity server.

The secure and verified peer-to-peer connection may be a direct link across any number of internet nodes that do not read or change the data being transmitted in any way. The data path is trusted, and both ends of the connection are verified as true and authentic sources. The data being transmitted is protected from third party interference. The data being transmitted may be bidirectionally verified as being received in full without corruption or loss of data, and/or bidirectionally verified such that the extent of any loss of data is known.

Data loss may be unavoidable in certain circumstances, however by verifying whenever data is lost, any lost data may be recovered or tracked such that missing data is correctly tracked in the digital twin.

The digital twin is a twin of the user, and possibly their MD(s). The digital twin may be updated in real-time. Real-time may include latency, e.g. introduced by processing sensor data, transmission time, encryption and decryption time. There may be a buffer of data to be transmitted, which may further introduce latency. Latency may occur in the MD, in the HCMS, and/or another device. Real-time may indicate that changes are transmitted in a relatively fast time-frame, such as within hours, preferably within minutes, and more preferably within seconds. Transmission time may be a balance between high speed and conserving energy.

The digital twin being a twin of the user may mean that any changes to the data of the user and/or their MD(s), including a new measurement or a changed parameter, is updated in the digital twin to also include this change (possibly in real-time). This may also mean that any changes to the digital twin, for example different operating parameters or a software update of the MD(s) in the digital twin, is updated in the real-world MD(s) (possibly in real-time).

This means that the digital twin is not a simulation of the MD, for all intents and purposes it is the MD. The digital twin provides a digital interface to more easily analyze and control the MD.

The parameters of the MD may be related to its function such as a sampling frequency, ranges used during measurements, or battery level. The parameters of the MD may not be related to regulatory information such as safety and drug information or insurance information.

While measurement data may be relevant to receive quickly, possibly in real-time (i.e. lagging only due to latency and buffering inherent to the transmission process), changes to the parameters of the MD(s) may be updated more seldom. For example, a change to the MD(s) in the digital twin may be copied to the real-world MD(s) during a periodic update window, e.g. during nighttime or biweekly.

This allows the MD(s) to be controlled remotely while maintaining strict control over who or what is allowed to make changes. The digital twin, being completely digital and possibly stored in a local and secure server of the MD manufacturer or a hospital, is much easier to control access to than the MD itself out in the wild. The MD itself may not even react to any control data or very few, such as turning the MD on and off and using a user interface on the device itself, and may only or primarily be controllable by altering the digital twin version of the MD.

Furthermore, the medical sensor data in the digital twin (which comprises data recently received by the digital twin as well as data stored in the digital twin, e.g. from earlier measurements) may be used by the HCMS or third parties interfacing with the HCMS to diagnose or monitor the patient. A medical professional interfacing with the HCMS, or the HCMS itself based on analysis of the data, may trigger certain actions in the HCMS, such as triggering an alarm or altering the MD of the digital twin to mitigate future danger or monitor something more closely. This automatic analysis of the data in the digital twin may be rudimentary, such as detecting when blood pressure or blood sugar exceeds a certain predetermined threshold. The automatic analysis of the data in the digital twin may be advanced, making use of external databases and advanced processing and algorithms not otherwise available to the MD.

The digital twin may be separated into a patient section, comprising measurements of the physiological condition of the user of the MD. The digital twin may further be separated into a medical device section, comprising current and historical operating parameters and conditions of the MD. Some parties may have access to only one section of the digital twin, or different levels of access to the different sections.

In this case, only the medical device section may be changed on the HCMS-side since medical technology at the time of this invention cannot change a person's medical condition remotely by sheer force of will. However, the medical device section may be changed to e.g. administer remote remedies such as an electric shock or a remote administration of medicament e.g. via remote injection, which will affect the patient itself, in turn affecting the patient section of the digital twin.

The patient section of the digital twin may be used to extract features from the available data, making use of more advanced processing power and algorithms than would be available on a remote MD concerned with conserving battery and transmitting measurements in real-time. This may include secondary medical indicators such as a likelihood of a seizure or heart attack in the future. Extracted features may also be stored in the digital twin, and their presence may trigger action by the HCMS or third parties interfacing with the HCMS, to e.g. trigger an alarms or alter the MD of the digital twin to mitigate future danger or monitor something more closely.

The user of the MD may be allowed access to the patient section, and may e.g. receive health updates and be able to access and control access to the medical data being collected. However, the user may be prevented from accessing at least parts of the medical device section, such that the operating parameters of the MD(s) are not able to be tampered with by the user.

It is noted that the medical data being collected is data for medical use sensed by sensor(s) of medical device(s). This is different from e.g. biometric data used for identification.

While third parties tampering with or accessing sensitive information is a real and relevant threat, a more likely danger to the usefulness of a remote study of a patient is the patient themselves messing with the MD in unintended and unexpected ways. By limiting control over the MD, even by the user, better results may be achieved.

The digital twin may further comprise additional information, such as contact information for the patient, primary physician and next of kin, as well as insurance data and other information relevant for parties accessing the digital twin. This allows the digital twin to be used as a medical record of the patient, ensuring an updated and accurate digital medical record.

The digital twin may be regarded as a specialized database, that comprises real-time updated medical data and historical medical data. The digital twin may store data according to a standard, e.g. using a medical picture archive system (PACS) and the data may further be standardized, e.g. formatted using DICOM.

Some of the data in the digital twin may be private and some may be public. This means that only some of the data may be accessible by third parties. Whether data is public or private may be coded into the data itself, based on where the data is stored in the digital twin, and/or based on how it is classified by the HCMS.

The patient-specific medical device and the health care management system may communicate data directly with each other via the peer-to-peer connection and through firewalls; a dedicated connectivity server may dynamically mediate the direct peer-to-peer connection by providing respective IP-addresses to the patient-specific medical device and the health care management system; and the data are not received by the connectivity server during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable MD, which may connect to different hubs using different connection types, e.g. switching from Wi-Fi to cellular communications (such as LTE) as the user leaves their home.

The connectivity server may be considered to assist in establishing the secured and verified peer-to-peer connection. For example, a network module, medical device, or personal server device may establish the secured and verified peer-to-peer connection by using the connectivity server.

The dedicated connectivity server enables hole-punching through firewalls so that the MD and HCMS may communicate directly with each other without other security measures causing interruptions or delay and with no intermediaries, including the connectivity server itself.

The dedicated connectivity server dynamically mediates the direct peer-to-peer connection by providing respective IP-addresses to the MD and the HCMS. In this way, the portable MD does not need a fixed IP address and can use any other unique ID instead such as a serial number or a MAC address. This enables the system to be more flexible and reliable, as well as more secure.

The connectivity server may function similar to the control server described in paragraphs [0058]-[0064] of US 2011/0055322 A1, which is hereby incorporated by reference.

The step of storing may further comprise storing parameters of the medical device at a time of measuring.

Such parameters may include battery status, location, position on or relative to the body and mode of use.

This enables more accurate tracking of data and allows the digital twin access to parameters of the medical device that may be further altered or optimized. Certain correlations or errors may be detected by comparing the measurements and the parameters that otherwise would be missed. For example, a measurement done while the battery was very low may be less reliable, and someone's heart rate increasing every time they drive on a specific road section may be relevant to get a more accurate picture of the patient's health condition.

Such parameters may be formatted in a similar manner as the measurement data. For example, if the measurement data uses a DICOM format a battery status may be formatted as a waveform with relatively low sampling frequency.

The method may further comprise a step of extracting features from the measurement data, wherein the step of storing further comprises storing the extracted features in the memory buffer.

Such features may be a sampling frequency extracted from the measurement data or a heart rate extracted from PPG data.

This step may be performed by the MD itself as it performs measurements and/or a separate device.

Features may be extracted from decrypted/historical data in the HCMS in a similar manner.

By extracting further features from the data, the digital twin will contain as much relevant data as possible.

The step of storing may further comprise storing an absolute time stamp for each measurement or set of measurements corresponding to the measurement/current data in the memory buffer.

Absolute time is synchronized, rooted or has a known drift compared to something absolute, such as true time (objectively correct), network time (shared by all devices in the same network), system time (shared by all devices in the same system, e.g. determined by a hub), or module time (being synchronized to system time with a known accuracy).

This means that regardless of how accurate the timekeeping is of e.g. an MD performing measurements, the time being recorded is known compared to something with predictably accurate timekeeping, which means that it can be compared with high precision to other absolute times, even if they have different drifts or use a different reference time.

The absolute time stamp may be true time information but does not have to be. The main benefit comes from the time stamp being absolute, i.e. independent of the MD itself and comparable with other absolute time stamps.

By using an absolute time stamp, each measurement or set of measurements may be comparable to other measurements, e.g. done by a different sensor that may or may not be a part of the digital twin and may or may not be accessible by the HCMS.

The time of the measurement may be recorded by the sensor and/or MD using an internal clock of the MD. The internal clock may be periodically synchronized with e.g. network time or server time from the HCMS.

An absolute time stamp of a set of measurements may have a time stamp of the first and last measurement of the set of measurements. The remaining time stamp(s) of any other measurement(s) of the set of measurements may thereby be calculated assuming the measurements are done with a fixed (sampling) frequency.

The step of storing may further comprise storing data in a standardized data format before being encrypted.

A standardized data format may e.g. be DICOM or HL7. By using a standardized data format for health data, relevant information may be stored in an efficient manner and other parties may access the data in the digital twin.

The data may be converted into a standard format and/or be received already in a standard format. The conversion may be performed by the MD or another device.

The method may further comprise a step of compressing data in the memory buffer in a lossless manner.

The method may further comprise a step of decompressing data in the health care management system.

Compression may be performed in any number of ways and algorithms known to a person skilled in the art. The compression being lossless means that no medical data are lost (during compression and decompression), which is beneficial as medical data may have strict requirements on being complete and authentic.

The decompression may occur after the data have been decrypted in any number of ways known to a person skilled in the art.

All data in the digital twin may be readable, i.e. decompressed and e.g. formatted to a standard such as DICOM. While the data are readable, there is still a layer of security, so they are not readable by unauthorized parties. It is assumed that storage of data for the digital twin is not a concern. Old data that is no longer relevant may be moved out of the digital twin and be compressed for storage in a digital medical archive/record.

The steps of (lossless) compressing and encrypting of data in the memory buffer may be performed in one combined operation.

By combining these operations, they may be performed in a more efficient manner. This is advantageous to keep latency low and save processing power.

Establishing a secured and verified peer-to-peer connection may comprise establishing a bidirectional system for verifying integrity of data being transmitted using the connection.

By verifying the integrity of data, both connected parties (the MD and the HCMS) may be sure that transmitted data are correctly and fully received by the other party. This may be of special importance for medical data, which may have strict requirements on being complete and authentic.

Such a system for verifying data integrity may comprise sending a confirmation that data have been received and that it is apparently uncorrupted and complete. This may comprise detecting whether received data are corrupted and/or incomplete (error detection), which may comprise any number of techniques for error detection known in the art, such as parity bit checks, or cyclic redundancy check (CRC) codes.

The system may send packages according to UDP or TCP protocols.

Sometimes errors may be corrected automatically, and sometimes retransmission may be required because the data are too corrupt to be corrected automatically. The autocorrection may use redundancy in the transmission to correct what may be missing. For example, a transmission may use frequency hopping that distributes data across different streams so that even if one stream is lost all data may be recovered using the other streams. Autocorrection may also or instead use two different types of communication at the same time for redundancy, however this is not always desirable because of higher power consumption and complexity.

In order to not lose data, the MD (or some other device in communication with the MD) may comprise a(n extended) memory buffer that stores data to be transmitted to the HCMS. In case of an error that would require retransmission to be corrected, the data in the memory buffer may be used. Since the HCMS long-term stores all data and changes, a memory buffer may not be needed for a retransmission from the HCMS.

If the MD is portable, transmission conditions may drastically change over time. For example, the user may go into a cellar with poor signal reception. In such a situation, the memory buffer may store measurement data and/or encrypted data as it is received/created, such that the stored data may be transmitted once conditions improve. Accordingly, storing the measurement data in a memory buffer and encrypting data in the memory buffer into encrypted data may comprise storing the measurement data and/or the encrypted data until the data are able to be transmitted (in a secure and verified manner).

In case the memory buffer fills up (runs out of available memory space) systems may be implemented to prioritise deleting specific types of data so that new (incoming in real-time) data may be stored.

For example, the memory buffer may be configured to delete the oldest data first (to make room for new data). Alternatively, or in conjunction with other priorities that may be weighed against each other, certain types of data may be prioritised to not be deleted, or to be deleted only under certain conditions, such as data comprising a specific type of measurement data or alarm/notification data.

The MD may have control over the priority system to implement, as the MD may know best what type(s) of data are important.

In case an error is detected that cannot be corrected (e.g. using autocorrection from redundancy or retransmission), it may be relevant to ensure that incorrect data is deleted. The intended use of the invention relates to medical data, which has different restrictions than e.g. streamed audio or video. Artifacts and delays may be accepted to a certain degree when streaming media, but errors in medical data may result in misdiagnosis. Therefore, losing data may be preferable to using data with errors. If data are lost, the digital twin may record which data have been lost.

Accordingly, establishing a secured and verified peer-to-peer connection may comprise error detection and/or ensuring that data with error(s) is deleted.

The MD may be configured to not transmit (via the secured and verified peer-to-peer connection) any data resulting from human input. The digital twin may be configured to reject any data resulting from human input. This is beneficial in that human error is avoided, such that the data in the HCMS may be especially trusted.

Establishing a secured and verified peer-to-peer connection may comprise establishing a system to prevent any third party from accessing and/or modifying the data being transmitted using the connection.

By preventing any third party from accessing and/or modifying the data being transmitted, both connected parties (the MD and the HCMS) may be sure that transmitted data are secure from being accessed or tampered with.

Preventing any third party from accessing and/or modifying the data being transmitted may comprise preventing nodes from reading data being transmitted as it is transmitted, ensuring that the communication between the connected parties is direct and peer-to-peer (e.g. by ensuring that each connected party has the other's IP address), and/or by encrypting and/or distributing transmission of data.

Updating the digital twin may comprise maintaining a copy of the measurement data and parameters in the medical device and the digital twin.

Maintaining a copy of the measurement data and parameters may comprise synchronizing the MD and HCMS in real-time using bidirectional communication (via the secured and verified peer-to-peer connection).

Maintaining a copy of the measurement data and parameters may comprise any change to the user and/or their MD(s), including a new measurement or a changed parameter, is updated in the digital twin to also include this change (in real-time or during a scheduled/periodic update). This may also mean that any changes to the digital twin, for example different operating parameters or a software update of the MD(s) in the digital twin, is updated in the real-world MD(s) (in real-time or during a scheduled/periodic update).

This ensures a correct and up-to-date digital twin to be used for studies and diagnosis, as well as reliable updates to the software and parameters of the MD(s).

The medical device may be configured to resist certain control data, whereby the method further comprises a step of controlling the medical device by controlling a corresponding (virtual) medical device in the digital twin, such that any change to the (virtual) medical device of the digital twin is replicated in the (real) medical device.

The corresponding medical device is a digital, virtual copy of the medical device in the real world.

Certain control data may be all data controlling the MD, all data comprising certain requests (such as data that want to alter the functionality of the MD or the transmission of measurement data/encrypted data), all data that do not contain certain types of data (such as security verification), all data not originating from the HCMS, all data from external devices, and/or all data except for buttons on the MD itself (or equivalent, such as buttons on a control device physically connected to the MD or a display with a touch-screen button).

Resisting control data may comprise ignoring any such data, or requiring additional verification (e.g. from the HCMS) to accept such data.

Resisting control data may further comprise resisting updates and/or comprising read-only memory blocks that store aspects of firmware not to be changed, such as program storage or security keys.

Resisting control data may further comprise a (physical or software-implemented) blocking mechanism that blocks access to a program storage of the MD. Resisting control data may further comprise a blocking mechanism that blocks access to one or more specific memory blocks of the MD.

It may be beneficial to prevent updates to the MD, as the MD may be certified to be used in medicine only with a specific firmware. In order to update the MD, it may be required to replace the MD or bring the MD to a licensed party with special security access and/or ability to replace e.g. the memory block(s) comprising the software to be updated.

At least parts of the HCMS may be updated and controlled separately from the MD and its certification requirements. The HCMS may comprise any number of interfacing devices, programs and third parties that make use of and/or control the digital twin. However, any changes that do not alter the MD in the digital twin may be considered to be unrelated to the MD itself and its certification. Further, changes that do alter the MD in the digital twin, such as specific algorithms that optimize parameters of the MD in the digital twin, may need to be certified for a specific version of the relevant software.

It may be further beneficial to prevent third parties from interfacing directly with the first aspect of the invention. For example, using a third party smartphone as a gateway to transmit the encrypted data to the health care management system via the secured and verified peer-to-peer connection may be problematic as any updates to that third party device (or any applications running on that device) may compromise the security of the invention.

This may further comprise synchronizing the medical device and the corresponding medical device of the digital twin so that any change medical device of the digital twin is (immediately) replicated in the medical device (in real-time). Any change to the medical device may also be replicated in the medical device of the digital twin.

In this way, the digital twin comprises a virtual copy of the medical device that for all intents and purposes are equivalent. As control data is resisted by the medical device, the medical device must be controlled through the HCMS and its digital twin, which improves security and reduces user error.

The method may further comprise an optimizing step of the health care management system optimizing at least one parameter of a corresponding (virtual) medical device in the digital twin and transmitting said optimized at least one parameter to the (real) medical device via the secured and verified peer-to-peer connection.

Transmitting said optimized at least one parameter to the medical device may comprise transmitting parameters already being used by the medical device. This may be relevant e.g. after a medical device has been reset or replaced.

This may further be relevant when the user has a set of medical devices that cooperate, such as a pair of hearing aids or a day-device and a night-device meant to be switched for each other depending on the time of day. Such sets of medical devices may be considered the same or different MDs in the digital twin.

The optimizing step of the HCMS optimizing at least one parameter of the MD may comprise the HCMS calibrating the MD.

The HCMS has access to more processing power and potentially external databases and algorithms, so using those additional resources to optimize the MD remotely improves the functionality of the MD without costing more power and memory for the MD and further means that the MD does not have to access external, untrusted sources directly to have additional data to base optimization on.

It is noted that optimization may go beyond simply verifying that certain parameters can be changed, the optimized parameters may be considered a specific choice to improve performance compared to the previous parameters.

The optimizing step may further be (semi-)automatic, whereby the HCMS periodically changes parameters of the MD (via the MD in the digital twin) independent from the rest of the data in the digital twin. This change may be based on human input or time.

For example, a study may want to record medical data using a first operational parameter for three days and a different operational parameter for the four following days. As another example, a lower sampling frequency may be used during night-time. As yet another example, a doctor may decide that a higher sampling frequency may be needed after reviewing the data in the digital twin from the last couple of days.

The optimizing step may comprise optimizing at least one parameter of the corresponding (virtual) medical device in the digital twin using historical decrypted data of said at least one sensor comprised in the (real) medical device and/or measurement data of at least one sensor not comprised in the (real or virtual) medical device and accessible by the digital twin.

The at least one sensor not comprised in the medical device and accessible by the digital twin may be a sensor of another medical device of the user communicating with the digital twin (with the same secured and verified peer-to-peer connection) in the same manner as the MD. The at least one sensor not comprised in the medical device and accessible by the digital twin may be a sensor used previously by the user (e.g. at a hospital or during a previous study), the measurement data having been added to the digital twin at some previous point in time. The at least one sensor not comprised in the medical device and accessible by the digital twin may be a sensor never used by the user, but having measurement data relevant to the optimizing step.

The optimizing step may use automation, image processing, AI, or other suitable data processing techniques on the relevant data to obtain at least one optimized parameter. Such techniques may not be feasible to use in the MD, but may be accessible to the HCMS.

The optimizing step may further comprise using a database of algorithms and/or parameters to optimize said at least one parameter.

The database may comprise optimization algorithms for parameters, and/or the database may comprise a lookup table of parameters that have been previous tested to be optimal (either alone or in combination with certain other parameters or certain environments or conditions of the MD).

Updating the digital twin may comprise verifying that the user of the medical device is the same user that corresponds to the digital twin in the health care management system.

Verifying that the user of the medical device is the correct user may comprise detecting any sudden and/or unexpected changes in the medical data, e.g. after a period of inactivity.

Verifying may comprise asking the user to input authentication or confirmation or using a biometric input. ,

If e.g. a couple living together use similar MDs, any mix-up may cause the data in the digital twin to be unreliable, therefore additional verification may be beneficial.

The method may further comprise an alarm/notification step of the health care management system detecting an event using current and/or historical decrypted data and notifying either the user by sending an alarm/notification to the medical device and/or notifying a third party by sending an alarm/notification to the third party.

Detecting an event may comprise applying a detection algorithm on the decrypted data, detecting a value of the decrypted data beyond predefined thresholds, detecting a sudden or significant change in the decrypted data, and/or detecting an event flag in the data (created by the MD or another device).

The third party may in this case be a medical professional, caretaker or relative. The alarm/notification to the third party may make use of external communication means, such as cellular, accessible by the HCMS.

According to a second aspect of the invention, a system for bidirectional communication between a patient-specific medical device with at least one sensor and a health care management system is provided. The system comprises: a patient-specific medical device comprising at least one sensor, the medical device being configured to store measurement data received from said at least one sensor in a memory buffer and encrypt the data into encrypted data; a health care management system configured to decrypt the encrypted data and maintain a digital twin corresponding to a user of the medical device comprising current and historical decrypted data of said medical device; and a network module configured to establish or use a secured and verified peer-to-peer connection between the patient-specific medical device and the health care management system; wherein the medical device is further configured to transmit the encrypted data to the health care management system via the secured and verified peer-to-peer connection (in real-time).

All the alternatives and advantages of the first aspect also apply to this aspect mutato mutandis.

The network module may be implemented in the medical device or another device. The network module may be implemented using processing circuitry programmed to perform certain functions.

The system may further comprise a dedicated connectivity server configured to dynamically mediate the peer-to-peer connection by providing respective IP-addresses to the patient-specific medical device and the health care management system; and to not receive data during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable MD, which may connect to different hubs using different connection types, e.g. switching from Wi-Fi to LTE as the user leaves their home.

According to a third aspect of the invention, a method for maintaining a patient-specific digital twin is provided. The method comprises steps of: receiving measurement data from at least one sensor sensing medical data from a specific patient; receiving at least one parameter of a medical device comprising said at least one sensor; storing the received measurement data in a patient-specific memory; storing said received at least one parameter in a device-specific memory; and continuously synchronizing the data in the device-specific memory with the medical device.

Continuously synchronizing the data in the device-specific memory is bidirectional, any change to the MD, including a new measurement or a changed parameter, is updated in the digital twin to also include this change in real-time. Any changes to the digital twin, for example different operating parameters or a software update of the MD in the digital twin, is updated in the real-world MD in real-time or during a scheduled/periodic update.

This means that the digital twin is not a simulation of the MD, for all intents and purposes it is the MD. The digital twin provides a digital interface to more easily analyze and control the MD.

The patient-specific memory may be synchronized in one direction, i.e. any changes to the user (measured by the MD) is updated in the digital twin to also include this change in real-time or during a scheduled/periodic update.

All data stored in the digital twin may be time-stamped. This may be considered a timeline in a database.

The time-stamp may be done by the MD or another device and received by the digital twin as a parameter to the received measurement data and/or as part of said at least one parameter of the MD.

Storing the received measurement data in a patient-specific memory may comprise storing a unique identifier of the sensor that sensed the measurement data.

This enables full reconstruction of the conditions under which the measurement was taken, in case further investigation would be beneficial or if the user alternates between two or more MDs (with equivalent functions).

The medical device and the patient-specific digital twin may communicate according to the method for bidirectional communication according to the first aspect of the invention.

Accordingly, all the alternatives and advantages of the first aspect also apply to this aspect mutato mutandis.

According to a fourth aspect of the invention, a method for bidirectional communication between a patient-specific medical device with at least one sensor and a health care management system is provided. The method comprises steps of: receiving measurement data from said at least one sensor; storing the measurement data (as current data) in a memory buffer; and applying a detection algorithm on the (current) measurement data in the memory buffer.

When the detection algorithm detects an event, the method further comprises steps of: generating event data; encrypting the event data into encrypted data; establishing a secured and verified peer-to-peer connection between the patient-specific medical device and the health care management system; transmitting the encrypted data to the health care management system via the secured and verified peer-to-peer connection; decrypting the encrypted data (into historical/decrypted data); updating a digital twin in the health care management system using the historical/decrypted data, the digital twin corresponding to a user of the medical device and comprising historical/decrypted data of said medical device; and notifying a third party by sending a notification/alarm based on the (decrypted) event data to the third party.

Event data comprises: data in the memory buffer related to the event detected by the detection algorithm; and an event type based on the event detected by the detection algorithm.

All the alternatives and advantages of the first aspect also apply to this aspect mutato mutandis.

An event type may be a heart attack, low blood-sugar, a seizure, a fall, or any other medical event. The event may be harmful to the user, especially if left unnoticed.

The detection algorithm may be configured to detect a value of the measurement data beyond predefined thresholds, detecting a sudden or significant change in the measurement data, and/or detecting an event flag in the data (created by the medical device (MD) or another device).

The third party may in this case be a medical professional, caretaker or relative. The user may also be notified. The notification may make use of external communication means, such as cellular, accessible by the MD, the health care management system (HCMS), or another device.

Updating the digital twin may comprise maintaining a copy of the measurement data and parameters in the medical device and in the digital twin.

Maintaining a copy of the measurement data and parameters may comprise synchronizing the MD and HCMS in real-time using bidirectional communication (via the secured and verified peer-to-peer connection).

Maintaining a copy of the measurement data and parameters may comprise any change to the user and/or their MD(s), including a new measurement or a changed parameter, is updated in the digital twin to also include this change in real-time or during a scheduled/periodic update. This may also mean that any changes to the digital twin, for example different operating parameters or a software update of the MD(s) in the digital twin, is updated in the real-world MD(s) in real-time or during a scheduled/periodic update.

It is noted that it may be especially beneficial to update data relating to event data in real-time rather than during a scheduled/periodic update.

The event data may trigger notifying a third party when it is created, when it is received by the HCMS, or both. Notifying a third party may be done in any number of ways known to a person skilled in the art, such as sending a text message, initiating an automated phone call, triggering a notification on an app (e.g. on a smartphone or computer), sending an email, or notifying an alarm center. The device or structure triggering the notification may use an internet connection, cellular connection, GDMSS, or any other suitable connection to reach the third party.

Notifying a third party may have a highest priority in terms of speed. This may cause the MD or other devices to use more energy than normal operation to transmit a notification faster than normal.

The memory buffer comprises the measurement data that the detection algorithm is applied to. The memory buffer may be configured to store data according to a first-in-first-out scheme. Alternatively, the memory buffer may be configured to delete some type of data first as more room is needed in the buffer, such as data not comprising any relevant information.

The detection algorithm may be applied to all or some data in the memory buffer, such as the most recent data. The detection algorithm may make use of prior data and/or a prior result of the detection algorithm, i.e. a consequence of the detection algorithm when applied to prior data.

For example, the detection algorithm may determine that, following certain current conditions, a specific future measurement may be cause for alarm. The detection algorithm may thereby store this specific future measurement as an event flag and be configured to detect an event if this event flag is encountered in future measurement data. This event flag may be deleted after a predetermined time or number of measurements.

This enables the detection algorithm to improve over time based on actual real-life data. The detection algorithm may also improve to better detect events that have personally affected the specific patient.

According to a fifth aspect of the invention, a method for bidirectional communication between a patient-specific medical device with at least one sensor and a health care management system via a Personal Server device, PS, is provided. The method comprises steps of: receiving measurement data to the PS from said at least one sensor; storing the measurement data (as current data) in a memory buffer of the PS; and applying a detection algorithm on the (current) measurement data in the memory buffer.

When the detection algorithm detects an event, the method further comprises steps of: generating event data; encrypting the event data into encrypted data; establishing a secured and verified peer-to-peer connection between the PS and the health care management system; transmitting the encrypted data to the health care management system via the secured and verified peer-to-peer connection; decrypting the encrypted data; updating a digital twin in the health care management system using the decrypted data, the digital twin corresponding to a user of the medical device and comprising historical decrypted data of said medical device; and notifying a third party by sending a notification/alarm based on the (decrypted) event data to the third party.

Event data comprises: data in the memory buffer related to the event detected by the detection algorithm; and an event type based on the event detected by the detection algorithm.

All the alternatives and advantages of the first aspect of the invention also apply to this aspect mutato mutandis.

The PS may be a PS according to the eighth aspect of the invention, with all alternatives and advantages mutato mutandis.

An event type may be a heart attack, low blood-sugar, a seizure, a fall, or any other medical event. The event may be harmful to the user, especially if left unnoticed.

The detection algorithm may be configured to detect a value of the measurement data beyond predefined thresholds, detecting a sudden or significant change in the measurement data, and/or detecting an event flag in the data (created by the MD, the PS, or another device).

The third party may in this case be a medical professional, caretaker or relative. The user may also be notified. The notification may make use of external communication means, such as cellular, accessible by the MD, the PS, the health care management system (HCMS), or another device.

Updating the digital twin may comprise maintaining a copy of the measurement data and parameters in the medical device and the digital twin.

Maintaining a copy of the measurement data and parameters may comprise synchronizing the MD and HCMS in real-time or during a scheduled/periodic update using bidirectional communication (via the secured and verified peer-to-peer connection of the PS).

Maintaining a copy of the measurement data and parameters may comprise any change to the user and/or their MD(s) or PS, including a new measurement or a changed parameter, is updated in the digital twin to also include this change in real-time or during a scheduled/periodic update. This may also mean that any changes to the digital twin, for example different operating parameters or a software update of the MD(s) or PS in the digital twin, is updated in the real-world MD(s) and/or PS in real-time or during a scheduled/periodic update.

It is noted that it may be especially beneficial to update data relating to event data in real-time rather than during a scheduled/periodic update.

The event data may trigger notifying a third party when it is created, when it is received by the HCMS, or both. Notifying a third party may be done in any number of ways known to a person skilled in the art, such as sending a text message or satellite emergency text message, initiating an automated phone call, triggering a notification on an app (e.g. on a smartphone or computer), or sending an email. The device or structure triggering the notification may use an internet connection, cellular connection, or any other suitable connection to reach the third party. The PS may use a connection of the MD, of its own, or of another device.

Notifying a third party may have a highest priority in terms of speed. This may cause the MD, PS or other devices to use more energy than normal operation to transmit a notification faster than normal.

The memory buffer comprises the (current) measurement data that the detection algorithm is applied to. The memory buffer may be configured to store data according to a first-in-first-out scheme. Alternatively, the memory buffer may be configured to delete some type of data first as more room is needed in the buffer, such as data not comprising any relevant information. The memory buffer may be configured to behave differently depending on the MD or sensor that measured the data.

The detection algorithm may be applied to all or some (current) data in the memory buffer, such as the most recent data. The detection algorithm may make use of prior data and/or a prior result of the detection algorithm, i.e. a consequence of the detection algorithm when applied to prior data.

According to a sixth aspect of the invention, a method for bidirectional communication between a patient-specific medical device with at least one sensor and a health care management system is provided. The method comprises steps of: receiving measurement data from said at least one sensor; storing the measurement data (as current data) in a memory buffer; encrypting (current) data in the memory buffer into encrypted data; establishing a secured and verified peer-to-peer connection between the patient-specific medical device and the health care management system; transmitting the encrypted data to the health care management system via the secured and verified peer-to-peer connection; decrypting the encrypted data (into historical/decrypted data); updating a digital twin in the health care management system using the historical/decrypted data (in real-time), the digital twin corresponding to a user of the medical device and comprising historical/decrypted data of said medical device; and applying a detection algorithm on the historical/decrypted data (and potentially other data in the digital twin).

When the detection algorithm detects an event, the method further comprises steps of: generating event data comprising: historical/decrypted data (and potentially other data in the digital twin) related to the event detected by the detection algorithm; and an event type based on the event detected by the detection algorithm; and notifying a third party by sending an alarm/notification based on the event data to the third party.

All the alternatives and advantages of the first aspect also apply to this aspect mutato mutandis.

An event type may be a heart attack, low blood-sugar, a seizure, a fall, or any other medical event. The event may be harmful to the user, especially if left unnoticed.

The detection algorithm may be configured to detect a value of the historical/decrypted data (and potentially other data in the digital twin) beyond predefined thresholds, detecting a sudden or significant change in the historical/decrypted data (and potentially other data in the digital twin), and/or detecting an event flag in the historical/decrypted data (created by the MD or another device).

The detection algorithm may compare the historical/decrypted data (and potentially other data in the digital twin) with other similar data accessible to the HCMS (e.g. via an external database). If certain correlations are found, e.g. between the historical/decrypted data (and potentially other data in the digital twin) and data leading to or resulting from an event.

The detection algorithm may apply deep learning algorithms to the historical/decrypted data (and potentially other data in the digital twin) to detect data leading to or resulting from an event.

The third party may in this case be a medical professional, caretaker or relative. The user may also be notified. The notification may make use of external communication means, such as cellular, accessible by the MD, the HCMS, or another device.

Updating the digital twin may comprise maintaining a copy of the measurement data and parameters in the medical device and the digital twin.

Maintaining a copy of the measurement data and parameters may comprise synchronizing the MD and HCMS in real-time or during a scheduled/periodic update using bidirectional communication (via the secured and verified peer-to-peer connection).

Maintaining the same measurement data and parameters may comprise any change to the user and/or their MD(s), including a new measurement or a changed parameter, is updated in the digital twin to also include this change in real-time or during a scheduled/periodic update. This may also mean that any changes to the digital twin, for example different operating parameters or a software update of the MD(s) in the digital twin, is updated in the real-world MD(s) in real-time or during a scheduled/periodic update.

It is noted that it may be especially beneficial to update data relating to event data in real-time rather than during a scheduled/periodic update.

The event data may trigger notifying a third party when it is created. Notifying a third party may be done in any number of ways known to a person skilled in the art, such as sending a text message, initiating an automated phone call, triggering a notification on an app (e.g. on a smartphone or computer), or sending an email. The HCMS may use an internet connection, cellular connection, or any other suitable connection to reach the third party.

Notifying a third party may have a highest priority in terms of speed.

The digital twin comprises the historical/decrypted data (and potentially other data) that the detection algorithm is applied to. The digital twin may be considered to have as much memory space as needed to store all historical/decrypted data (and potentially other data).

The detection algorithm may be applied to all or some data in the digital twin, such as the most recent data. The detection algorithm may make use of prior data and/or a prior result of the detection algorithm, i.e. a consequence of the detection algorithm previously applied.

For example, the detection algorithm may determine that, following certain current conditions, a specific future measurement may be cause for alarm. The detection algorithm may thereby store this specific future measurement as an event flag and be configured to detect an event if this event flag is encountered in future decrypted data. This event flag may be deleted after a predetermined time or number of measurements.

According to a seventh aspect of the invention, a method for bidirectional communication between a patient-specific medical device with at least one sensor and a health care management system via a Personal Server device, PS, is provided. The method comprises steps of: receiving measurement data to the PS from said at least one sensor; storing the measurement data (as current data) in a memory buffer of the PS; and encrypting (current) data in the memory buffer into encrypted data; establishing a secured and verified peer-to-peer connection between the PS and the health care management system; transmitting the encrypted data to the health care management system via the secured and verified peer-to-peer connection; decrypting the encrypted data (into historical/decrypted data); updating a digital twin in the health care management system using the historical/decrypted data (in real-time), the digital twin corresponding to a user of the medical device and comprising historical/decrypted data of said medical device; and applying a detection algorithm on the historical/decrypted data (and potentially other data in the digital twin).

When the detection algorithm detects an event, the method further comprises steps of: generating event data comprising: historical/decrypted data (and potentially other data in the digital twin) related to the event detected by the detection algorithm; and an event type based on the event detected by the detection algorithm; and notifying a third party by sending a notification/alarm based on the event data to the third party.

All the alternatives and advantages of the first aspect also apply to this aspect mutato mutandis.

The PS may be a PS according to the eighth aspect of the invention, with all alternatives and advantages mutato mutandis.

An event type may be a heart attack, low blood-sugar, a seizure, a fall, or any other medical event. The event may be harmful to the user, especially if left unnoticed.

The detection algorithm may be configured to detect a value of the historical/decrypted data (and potentially other data in the digital twin) beyond predefined thresholds, detecting a sudden or significant change in the historical/decrypted data (and potentially other data in the digital twin), and/or detecting an event flag in the historical/decrypted data (created by the MD, the PS or another device).

The detection algorithm may compare the historical/decrypted data (and potentially other data in the digital twin) with other similar data accessible to the HCMS (e.g. via an external database). If certain correlations are found, e.g. between the historical/decrypted data (and potentially other data in the digital twin) and data leading to or resulting from an event.

The detection algorithm may apply deep learning algorithms to the historical/decrypted data (and potentially other data in the digital twin) to detect data leading to or resulting from an event.

The third party may in this case be a medical professional, caretaker or relative. The user may also be notified. The notification may make use of external communication means, such as cellular, accessible by the MD, the PS, the HCMS, or another device.

Updating the digital twin may comprise maintaining a copy of the measurement data and parameters in the medical device and the digital twin.

Maintaining a copy of the measurement data and parameters may comprise synchronizing the MD and HCMS in real-time or during a scheduled/periodic update using bidirectional communication (via the secured and verified peer-to-peer connection of the PS).

Maintaining a copy of the measurement data and parameters may comprise any change to the user and/or their MD(s) or PS, including a new measurement or a changed parameter, is updated in the digital twin to also include this change in real-time or during a scheduled/periodic update. This may also mean that any changes to the digital twin, for example different operating parameters or a software update of the MD(s) or PS in the digital twin, is updated in the real-world MD(s) and/or PS in real-time or during a scheduled/periodic update.

It is noted that it may be especially beneficial to update data relating to event data in real-time rather than during a scheduled/periodic update.

The event data may trigger notifying a third party when it is created. Notifying a third party may be done in any number of ways known to a person skilled in the art, such as sending a text message or satellite emergency text message, initiating an automated phone call, triggering a notification on an app (e.g. on a smartphone or computer), or sending an email. The HCMS may use an internet connection, cellular connection, or any other suitable connection to reach the third party.

Notifying a third party may have a highest priority in terms of speed.

The digital twin comprises the historical/decrypted data (and potentially other data) that the detection algorithm is applied to. The digital twin may be considered to have as much memory space as needed to store all historical/decrypted data (and potentially other data).

The detection algorithm may be applied to all or some data in the digital twin, such as the most recent data. The detection algorithm may make use of prior data and/or a prior result of the detection algorithm, i.e. a consequence of the detection algorithm previously applied.

The patient-specific medical device and the health care management system may communicate data directly with each other via the peer-to-peer connection and through firewalls; a dedicated connectivity server may dynamically mediate the direct peer-to-peer connection by providing respective IP-addresses to the patient-specific medical device and the health care management system; and the data may not be received by the connectivity server during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable MD, which may connect to different hubs using different connection types, e.g. switching from Wi-Fi to LTE as the user leaves their home.

The dedicated connectivity server enables hole-punching through firewalls so that the MD and HCMS may communicate directly with each other without other security measures causing interruptions or delay and with no intermediaries, including the connectivity server itself.

The dedicated connectivity server dynamically mediates the direct peer-to-peer connection by providing respective IP-addresses to the MD and the HCMS. In this way, the portable MD does not need a fixed IP address and can use any other unique ID instead such as a serial number or a MAC address. This enables the system to be more flexible and reliable, as well as more secure.

Such a connectivity server may function as any other connectivity server mentioned in relation to other aspects of the invention mutatis mutandis.

The PS and the health care management system may communicate data directly with each other via the peer-to-peer connection and through firewalls; a dedicated connectivity server may dynamically mediate the direct peer-to-peer connection by providing respective IP-addresses to the PS and the health care management system; and the data may not be received by the connectivity server during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable PS, which may connect to different hubs using different connection types, e.g. switching from Wi-Fi to LTE as the user leaves their home.

The dedicated connectivity server enables hole-punching through firewalls so that the PS and HCMS may communicate directly with each other without other security measures causing interruptions or delay and with no intermediaries, including the connectivity server itself.

The dedicated connectivity server dynamically mediates the direct peer-to-peer connection by providing respective IP-addresses to the PS and the HCMS. In this way, the PS does not need a fixed IP address and can use any other unique ID instead such as a serial number or a MAC address. This enables the system to be more flexible and reliable, as well as more secure.

Such a connectivity server may function as any other connectivity server mentioned in relation to other aspects of the invention mutatis mutandis.

The method may further comprise a step after generating event data of updating the digital twin with the event data.

The digital twin is continuously updated with current decrypted data. By also updating the digital twin with event data, the event type and the data related to the event (i.e. data causing and/or correlating to the event or being measured around the time of the event) will be in the digital twin. This data may be used by third parties or the detection algorithm to improve the detection algorithm and to better understand events and the health of the user. Updating the digital twin with the event data may be done in real-time.

The method may further comprise a step of when the detection algorithm detects an event, notifying the user and providing instructions based on the event data.

Notifying the user may comprise an indication on the MD or PS such as a blinking light, a sound, or a screen pop-up (the screen may be a part of the MD, the PS or the user's smartphone). Notifying the user may comprise the MD, PS, or HCMS sending a notification to the user in a similar manner as notifying a third party previously discussed.

Providing instructions may comprise notifying that help is on its way, to perform certain tasks such as lying down, taking medication or taking a shot of adrenaline, or to request help from people close to the user.

The event data may further comprise an absolute time stamp of the event, position data of the user, duration and intensity of the event, and/or parameters of the medical device at the time of the event.

All this information may be useful for determining which third party to contact, what caused the event, how to best help the user in their current situation, and how to proceed helping the user moving forward. This data may be used to improve the detection algorithm and help an ambulance find the user.

The detection algorithm may be configured to detect a change in sensor data, a sudden presence or absence of data or pattern in the sensor data, and/or an event flag.

The detection algorithm may be configured to detect a value of the sensor data beyond predefined thresholds or a sudden or significant change in the sensor data.

The sensor data may be measurement data or decrypted data comprising measurement data. An event flag may be created by the MD, the PS, the HCMS, or another device to ensure the detection algorithm detects something specific.

The method may further comprise a step of updating the detection algorithm based on historical/decrypted data of said medical device and/or a database of algorithms.

This updating step may comprise using previous event data to improve the detection algorithm. The previous event data may be from the user or another user of a similar MD.

Using an external database and/or historical, personalized data may make the detection algorithm better at detecting events to be detected and avoiding detecting false events.

As the HCMS continues to collect historical/decrypted data in the digital twin, the detection algorithm may be continuously improved by training on user-specific and/or MD-specific data.

Notifying a third party may comprise the health care management system selecting a third party to notify based on contact data of the user of the digital twin and sending a notification/alarm to the third party.

The contact data may be stored in the digital twin or some other database accessible by the HCMS. The third party may be a caretaker or family member that has agreed to have their contact data made available for this purpose.

The third party notified may be based on the event data.

This may improve the response to an event and get the user the best possible help when needed.

For example, if the user has a seizure, it may be less relevant to contact a doctor and more relevant to contact someone close to the user such as a caretaker or family member. The position of the user may be used to contact the family member closest to the user at the time.

As another example, if the user is having a severe heart attack, it may be important to contact emergency services to get an ambulance to the user as fast as possible. The user's position and the severity of the event may be provided to emergency services to improve the response.

As another example, if the user's blood sugar is hitting certain thresholds, it may be relevant for the user's doctor to receive that information, but not in real-time, but rather as a summary of all such instances over one or more days.

A virtual channel may be assigned for each data or group of data being transmitted via the secured and verified peer-to-peer connection, wherein each virtual channel has a relative priority and data comprising event data has a highest priority.

A virtual channel may be assigned for each data or group of data being transmitted via the secured and verified peer-to-peer connection, wherein each virtual channel has a relative priority and data notifying a third party has a highest priority.

Such virtual channels may be used to more efficiently transmit different types of data, such as data coming from a specific sensor, having a specific size range, or having a specific type (e.g. waveform or heatmap).

By each virtual channel having a relative priority, data that are more time-sensitive (such as event data triggering a notification to a third party or the notification itself) may be transmitted faster.

The detection algorithm may further be configured to determine that, based on the sensor data being analyzed, a specific, future range or set of sensor data may be more likely to be relevant for an event; and to store said specific, future range or set of sensor data as an event flag to be detected in the future.

This may enable faster reaction to an event about to happen or that is currently happening.

According to an eighth aspect of the invention, a personal server device (PS) for facilitating (a secured and verified) peer-to-peer bidirectional connection between a one or more patient-specific medical devices with at least one sensor and health care management system is provided.

The personal server device comprises: a first wireless communication radio for establishing or using a first wireless connection; a second wireless communication radio for establishing or using a second wireless connection; a processing circuit; and a memory buffer.

The personal server device is configured to: establish or use a local wireless communication with said one or more patient-specific medical devices via the first wireless connection for transmitting encrypted data to and from said one or more patient-specific medical devices; establish or use a secured and verified peer-to-peer connection to the health care management system via the second wireless connection for transmitting encrypted data to and from the health care management system; transmit encrypted data from said one or more patient-specific medical devices to the health care management system and from the health care management system to said one or more patient-specific medical devices using the first and second wireless connections; execute error detection on the first and second wireless connections; and buffer the encrypted data received from said one or more patient-specific medical devices to be transmitted to the health care management system until the encrypted data are safely received by the health care management system.

The PS may be configured to transmit any data received by the medical device (MD) to the health care management system (HCMS) in real-time. The PS may be configured to transmit any data received by the HCMS to the MD in real-time.

All the alternatives and advantages of the first aspect also apply to this aspect mutato mutandis. The PS may be regarded as a part of the MD or another device for this purpose. The PS having a first wired communication with said one or more patient-specific medical devices may be considered to be a part of the MD.

The PS may be a dedicated device or a personal device such as a smartphone with software implementing the invention.

The first and second wireless communication radios may be integrated in a single circuit or separate circuits. Each radio should be considered as a separate radio, even if e.g. it's the same circuit switching between different states.

The local wireless communication transmits encrypted data and may be secure and verified. While the local communication is local and thereby harder for third parties to interfere with, medical data may be considered so sensitive that it should always be encrypted before being transmitted.

Error detection may comprise detecting whether received data are corrupted and/or incomplete, which may comprise any number of techniques for error detection known in the art, such as parity bit checks, or cyclic redundancy check (CRC) codes.

The system may send packages according to UDP or TCP protocols.

Sometimes errors may be corrected automatically, and sometimes retransmission may be required because the data are too corrupt to be corrected automatically. The autocorrection may use redundancy in the transmission to correct what may be missing. For example, Bluetooth may use frequency hopping that distributes data across different streams so that even if one stream is lost all data may be recovered using the other streams. Autocorrection may also or instead use two different types of communication at the same time for redundancy, however this is not always desirable because of higher power consumption and complexity. Error detection is a balance between latency, power consumption and accuracy.

In order to not lose data, the PS (or some other device in communication with the PS, such as the MD) may comprise a(n extended) memory buffer that stores data to be transmitted to the HCMS. In case of an error that would require retransmission to be corrected, the data in the memory buffer may be used. Since the HCMS long-term stores all data and changes, a memory buffer may not be needed for a retransmission from the HCMS.

If the PS is portable, transmission conditions may drastically change over time. For example, the user may go into a cellar with poor signal reception. In such a situation, the memory buffer may store measurement data and/or encrypted data (as current data) as it is received/created, such that the stored data may be transmitted once conditions improve. Accordingly, storing the measurement data in a memory buffer and encrypting data in the memory buffer into encrypted data may comprise storing the measurement data and/or the encrypted data until the data are able to be transmitted (in a secure and verified manner).

In case the memory buffer fills up (runs out of available memory space) systems may be implemented to prioritise deleting specific types of data so that new (incoming in real-time) data may be stored.

For example, the memory buffer may be configured to delete the oldest data first (to make room for new data). Alternatively, or in conjunction with other priorities that may be weighed against each other, certain types of data may be prioritised to not be deleted, or to be deleted only under certain conditions, such as data comprising a specific type of measurement data or alarm/notification data.

Each MD may have control over the priority system to implement, as the MD may know best what type(s) of data are important.

In case an error is detected that cannot be corrected (e.g. using autocorrection from redundancy or retransmission), it may be relevant to ensure that incorrect data is deleted. The intended use of the invention relates to medical data, which has different restrictions than e.g. streamed audio or video. Artifacts and delays may be accepted to a certain degree when streaming media, but errors in medical data may result in misdiagnosis. Therefore, losing data may be preferable to using data with errors.

Accordingly, establishing or using a secured and verified peer-to-peer connection may comprise error detection and ensuring that data with error(s) are deleted.

The PS may be configured to not transmit (via the secured and verified peer-to-peer connection) any data resulting from human input. The digital twin may be configured to reject any data resulting from human input. This is beneficial in that human error is avoided, such that the data in the HCMS may be especially trusted.

The first and second wireless connections may be used to create a peer-to-peer connection between the PS and the health care management system such that they may communicate data directly with each other via the peer-to-peer connection and through firewalls; a dedicated connectivity server in the cloud may dynamically mediate the direct peer-to-peer connection by providing respective IP-addresses to the PS and the health care management system; and the data may not be received by the connectivity server during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable PS, which may connect to different hubs using different connection types, e.g. switching from Wi-Fi to LTE as the user leaves their home.

The dedicated connectivity server enables hole-punching through firewalls so that the PS and HCMS may communicate directly with each other without other security measures causing interruptions or delay and with no intermediaries, including the connectivity server itself.

The dedicated connectivity server dynamically mediates the direct peer-to-peer connection by providing respective IP-addresses to the PS and the HCMS. In this way, the portable PS does not need a fixed IP address and can use any other unique ID instead such as a serial number or a MAC address. This enables the system to be more flexible and reliable, as well as more secure.

Each of said one or more patient-specific medical devices may use the same peer-to-peer connection between the PS and the health care management system with separate virtual channels for each patient-specific medical device.

This may simplify the connection between the PS and HCMS and further enable more efficient and secure transmission.

Each of said one or more patient-specific medical devices may use a different peer-to-peer connection between the PS and the health care management system for each patient-specific medical device.

This may enable more advanced systems of MDs and HCMS with the PS as a mediator. For example, each MD may have a separate HCMS that cannot share the same peer-to-peer connection. However, by sharing the same PS, each HCMS may have access to (parts of) each MD. For example, perhaps an HCMS associated with a specific MD has access to measurements and parameters of that MD via the PS, and also has access to measurements of other MDs connected to the same PS.

This is a matter of security to be decided upon for each specific implementation of the inventive concept, but options exist should the manufacturer and systems manager want to use them.

Different peer-to-peer connections may further enhance security for the data of each of said one or more patient-specific medical devices. This is because each different peer-to-peer connection is secure and may be controlled by a specific MD/HCMS pair (via the PS).

Each different peer-to-peer connection may use a same connectivity server for mediating/establishing the peer-to-peer connection.

The device may be further configured to buffer the encrypted data received from said one or more patient-specific medical devices to be transmitted to the health care management system until receiving an acknowledgement from the health care management system that the encrypted data have been received (in full and correctly) by the health care management system.

Receiving an acknowledgement may be considered verifying the integrity of the data being transmitted.

By verifying the integrity of data, both connected parties (the PS and the HCMS) may be sure that transmitted data is correctly and fully received by the other party. This may be of special importance for medical data, which may have strict requirements on being complete and authentic.

Such a system for verifying data integrity may comprise sending a confirmation that data have been received and that it is apparently uncorrupted and complete. This may comprise detecting whether received data are corrupted and/or incomplete (error detection), which may comprise any number of techniques for error detection known in the art, such as parity bits, or cyclic redundancy check (CRC) codes.

In order to not lose data, the PS may comprise a(n extended) memory buffer that stores data to be transmitted to the HCMS. In case of an error that would require retransmission to be corrected, the data in the memory buffer may be used. Since the HCMS long-term stores all data and changes, a memory buffer may not be needed for a retransmission from the HCMS.

If the PS is portable, transmission conditions may drastically change over time. For example, the user may go into a cellar with poor signal reception. In such a situation, the memory buffer may store encrypted data as they are received, such that the stored data may be transmitted once conditions improve. Accordingly, storing the encrypted data in a memory buffer may comprise storing the encrypted data until the data are able to be transmitted (in a secure and verified manner).

Sometimes errors may be corrected automatically, and sometimes retransmission may be required because the data are too corrupt to be corrected automatically. The autocorrection may use redundancy in the transmission to correct what may be missing. For example, a transmission may use frequency hopping that distributes data across different streams so that even if one stream is lost all data may be recovered using the other streams. Autocorrection may also or instead use two different types of communication at the same time for redundancy, however this is not always desirable because of higher power consumption and complexity.

In case an error is detected that cannot be corrected (e.g. using autocorrection from redundancy or retransmission), it may be relevant to ensure that incorrect data is deleted. The intended use of the invention relates to medical data, which has different restrictions than e.g. streamed audio or video. Artifacts and delays may be accepted to a certain degree when streaming media, but errors in medical data may result in misdiagnosis. Therefore, losing data may be preferable to using data with errors.

When receiving encrypted data, the PS may apply a logical scheme to determine whether the data are correctly received and replies with a confirmation receipt.

When receiving encrypted data, the HCMS may apply a logical scheme to determine whether the data are correctly received and reply with a confirmation receipt.

This enables verifying the integrity of data being transmitted.

The logical scheme may be based on any number of techniques for error detection known in the art, such as parity bits, or cyclic redundancy check (CRC) codes.

The data being correctly received may comprise that the data are authentic, with no losses, errors, or corrupted data.

The device may be further configured to periodically synchronize the internal clocks of said one or more patient-specific medical devices.

By the PS synchronizing the MDs, their measurements may be more accurately comparable with each other, e.g. in the digital twin.

Any drift in internal clocks of the MDs may be compensated for by the periodic synchronization.

The time used to synchronize may be network time acquired via the second wireless connection, server time from the health care management system and/or precise time information from a GPS-receiver.

Server time and precise time information may be considered an absolute time standard that allows absolute time stamps to be comparable to any other absolute time stamp.

Precise time information from a GPS satellite may be considered true time.

By using an absolute time standard, each measurement or set of measurements may be comparable to any other measurements, e.g. done by a different sensor that may or may not be a part of the digital twin and may or may not be accessible by the HCMS.

The device may be further configured to provide position data of the PS to said one or more patient-specific medical devices and/or to the health care management system.

Position data may be useful for many different uses. For example, the MDs may act differently at a user's home or when they are travelling. The HCMS may find correlation between the location of the user and the measurement data of the MDs. Any potentially harmful event may be responded to faster if the position or the user is known.

The device may further comprise a GPS receiver configured to provide GPS position data, GNSS, and/or precise time information.

GPS position data is highly accurate and GNSS and precise time information may be useful for MDs. However, using a GPS receiver may require a relatively high amount of energy and take up valuable space in an MD. By the PS comprising a GPS receiver, each MD may make use of it without spending energy and space on a GPS receiver. Additionally, only one GPS receiver would be necessary instead of one per MD.

The GPS receiver and the second wireless communication radio may use a common antenna.

Such a configuration may save space and be more efficient.

The GPS receiver may further comprise a dedicated antenna.

Such a configuration may have a better performance (more powerful, more energy efficient) than other alternatives.

The first wireless communication radio may be a Bluetooth Low Energy, BLE, radio or a Thread radio.

These alternatives are energy-efficient and have good transmission characteristics at most intended ranges of operation.

The second wireless communication radio is a cellular radio (e.g. 4G, 5G, LoRaWAN, LPWAN, NB-loT, LTE or LTE-M), a Thread radio or a Wi-Fi radio.

Other suitable long-range wireless communication radios are possible, some of which will likely be developed after the drafting of this document.

These alternatives are energy-efficient, have a high bandwidth and capacity and have good transmission characteristics at most intended ranges of operation.

The device may be further configured to assign a virtual channel for each data or group of data being transmitted between said patient-specific medical devices and the health care management system, wherein virtual channels have a priority relative to each other.

Virtual channels may further enhance security and efficiency for the data of each data or group of data.

A group of data may be all data from a specific MD, all data comprising measurements from a specific sensor or of a specific type (e.g. waveform or image).

By each virtual channel having a relative priority, data that are more time-sensitive (such as event data triggering a notification to a third party or the notification itself) may be transmitted faster. This may comprise skipping ahead in the buffer or queue or using more energy to transmit in a different manner.

Virtual channels may extend all the way from the HCMS to the MD, or just between the HCMS and the PS or between the PS and the MD.

The device may be further configured to group data packets to be transmitted according to a transmission speed priority and/or transmission efficiency.

The data packets may be encrypted or not as they are grouped.

The grouping may be according to predefined rules, such as grouping packets so the groups are all within a specific size range, or grouping packets so that similar data are in the same group.

The grouping may group packets with a similar priority together, and the group with a highest priority may be transmitted first or as soon as possible.

Such grouping may improve efficiency and enable priority management.

The device may be further configured to group data packets to be transmitted based on the connection type to be used (and perhaps recent history of using that connection type).

The grouping may change depending on the availability and stability of different connection types. For example, the grouping may make groups of different sizes depending on if they are to be sent via Bluetooth, Wi-Fi, or LTE and how reliable the different options are deemed to be.

The connection type may further influence how much redundancy is used when grouping data packets.

By changing the grouping based on connection type, different bandwidths are better optimized and risk is mitigated.

Optimizing grouping of packets may be considered a balance between packages being big, taking time and having a bigger risk if they are lost and being small, costing too much overhead.

If a retransmission occurs due to a lost or corrupted packet, this may further affect how data packets are grouped to be transmitted, e.g. using smaller packages for some next predefined number of packages sent using that connection.

By optimizing how data packets are transmitted, each of the first and second wireless connections may be used more efficiently and accurately despite sending information across two different connections.

The device may be further configured to decrypt the encrypted data received by said one or more patient-specific medical devices and re-encrypt the data before transmitting the re-encrypted data to the health care management system.

By the PS being able to analyze the data, the transmission process may be made more efficient. For example, a more powerful encryption and/or compression may be able to be used than what is available to an MD. The encryption may further be better optimized to the specific connection type to be used, as this may only be known by the PS.

The device may be further configured to extract features from the decrypted data of said one or more patient-specific medical devices, to encrypt the extracted features, and to transmit the encrypted extracted features to the health care management system.

Such feature extraction may be a sampling frequency extracted from the measurement data or a heart rate extracted from PPG data and may include secondary medical indicators such as a likelihood of a seizure or heart attack in the future. Extracted features may also be stored in the digital twin, and their presence may trigger action by the HCMS or the PS, to e.g. trigger notifications/alarms or send control data to the MD to mitigate future danger or monitor something more closely.

The device may be adapted to be portable.

It may also be wearable and/or permanently wearable (i.e. implanted).

This enables real-time data collection to be done without interference with daily life. Many studies in the prior art are performed in an unrealistic environment, e.g. in a hospital. This causes the data to be obtained to be less reliable and more homogenized than if the patient was allowed to live their life like normal during the study. Portability thereby enables a more realistic and improved data collection.

The PS being portable also makes better use of many other (potential) features of the device. The concept of real-time collection of data is significantly harder to do reliably and securely in a shifting environment, but the PS has been envisioned with this in mind.

The device may be further configured to delete data according to logic related to each of said patient-specific medical devices if the buffer is full.

In case the memory buffer fills up (runs out of available memory space) systems may be implemented to prioritise deleting specific types of data so that new (incoming in real-time) data may be stored.

Said logic may be to delete oldest data or newest data first.

The memory buffer may be configured to delete the oldest data first (to make room for new data) or the newest data first (as new data will come when the buffer is cleared again).

Alternatively, or in conjunction with other priorities that may be weighed against each other, certain types of data may be prioritised to not be deleted, or to be deleted only under certain conditions, such as data comprising a specific type of measurement data or alarm/notification data.

Each MD may have control over the priority system to implement, as the MDs may know best what type(s) of data are important. Each medical device may have several different priorities, for example if the same sensor measures different things, an MD comprises several sensors, or a derived feature from a sensor is to have a different priority than the sensor data itself.

Each of said patient-specific medical devices may have a relative priority.

For example, if one MD is deemed most important, data from all other MDs may be deleted first.

To determine which MD is most important, each MD may have a default priority value, however this value may be changed when installing a new MD, the PS or updating the HCMS.

The device may further comprise at least one button, wherein said at least one button is configured to execute different functions depending on what patient-specific medical devices are in communication with the PS.

The button may be a physical actuator, or equivalent, such as buttons on a control device connected to the PS or a display with a touch-screen button.

This may enable the user to trigger actions such as starting a measurement or a transmission.

The device may further comprise a Satellite Emergency SOS radio.

An SOS radio has limited bandwidth but is globally available. This may be relevant to e.g. ensure an alarm/notification is sent even if other connections are not available, such as the notifications discussed in relation to aspects five and seven of the invention.

The device may further comprise an electrically conductive or absorbent screen that reduces the rate at which energy from the electromagnetic field generated by the device's transmitting circuits is absorbed by the patient's body (Specific Absorption Rate, SAR).

By reducing the SAR, the patient may be safer to use the device close to their body and/or for a long(er) time.

According to a ninth aspect of the invention, a system for bidirectional communication between one or more patient-specific medical devices with at least one sensor and a health care management system is provided.

The system comprises: one or more patient-specific medical devices comprising at least one sensor, each medical device being configured to store measurement data received from said at least one sensor (as current data) in a memory buffer and encrypt the (current/measurement) data into encrypted data; a health care management system configured to decrypt the encrypted data (into historical/decrypted data) and maintain a digital twin (in real-time) corresponding to a user of said one or more patient-specific medical devices comprising historical/decrypted data of said one or more patient-specific medical devices; and a personal server device (PS).

The PS comprises: a first wireless communication radio for establishing or using a first wireless connection; a second wireless communication radio for establishing or using a second wireless connection; a processing circuit; and a memory buffer.

The PS is configured to: establish or use a local wireless communication with said one or more patient-specific medical devices via the first wireless connection for transmitting encrypted data to and from said one or more patient-specific medical devices; establish or use a secured and verified peer-to-peer connection to the health care management system via the second wireless connection for transmitting encrypted data to and from the health care management system; and transmit encrypted data from said one or more patient-specific medical devices to the health care management system and from the health care management system to said one or more patient-specific medical devices using the first and second wireless connections.

All the alternatives and advantages of the second aspect also apply to this aspect mutato mutandis.

The digital twin may be maintained by periodically updating the digital twin to comprise a copy of the measurement data (i.e. historical/decrypted data corresponding to current/measurement data) and parameters as in said one or more patient-specific medical device and the digital twin.

Maintaining a copy of the measurement data and parameters may comprise synchronizing the MD(s) and HCMS in real-time using bidirectional communication (via the secured and verified peer-to-peer connection).

Maintaining a copy of the measurement data and parameters may comprise any change to the user and/or their MD(s), including a new measurement or a changed parameter, is updated in the digital twin to also include this change in real-time (i.e. the digital twin is updated in real-time as measurements are sensed by said at least one sensor) or during a scheduled/periodic update. This may also mean that any changes to the digital twin, for example different operating parameters or a software update of the MD(s) in the digital twin, are updated in the real-world MD(s) in real-time or during a scheduled/periodic update.

This ensures a correct and up-to-date digital twin to be used for studies and diagnosis, as well as reliable updates to the software and parameters of the MD(s).

The system may further comprise a dedicated connectivity server configured to dynamically mediate the peer-to-peer connection by providing respective IP-addresses to the PS and the health care management system; and to not receive data during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable MD, which may connect to different hubs using different connection types, e.g. switching from Wi-Fi to LTE as the user leaves their home.

The PS may be configured according to the eighth aspect of the invention.

The same alternatives and benefits apply mutatis mutandis.

According to a tenth aspect of the invention, a portable personal server device (PS) for facilitating a secured and verified peer-to-peer bidirectional connection between one or more patient-specific medical devices with at least one sensor and a health care management system (HCMS) is provided. The portable personal server device comprises: a first wireless communication radio for using a local wireless connection; a second wireless communication radio for using a secured and verified peer-to-peer connection; a primary memory buffer; and a memory processing circuit. The personal server device is configured to:use the local wireless connection with said one or more patient-specific medical devices via the first wireless communication radio for transmitting measurement data to and from said one or more patient-specific medical devices; use the secured and verified peer-to-peer connection with the health care management system via the second wireless communication radio for transmitting measurement data to and from the health care management system; and receive measurement data from at least one sensor, the measurement data comprising a time stamp corresponding to a time of measurement. The memory processing circuit is configured to: assign a first priority to the measurement data proportional to and increasing with an age of the time stamp; assign a second priority to the measurement data proportional to and decreasing with the age of the time stamp; and store the measurement data in the primary memory buffer. The personal server device is further configured to transmit the measurement data to the health care management system in order according to the second priority. The memory processing circuit is further configured to: delete the transmitted measurement data after receiving confirmation that the measurement data have been successfully transmitted; and delete non-transmitted data in order according to the first priority when storage capacity in the primary memory buffer reaches a predetermined limit.

The portable personal server device may further receive parameter data from the health care management system to facilitate synchronization with the digital twin. This parameter data may be assigned a fourth priority proportional to and increasing with an age of its time stamp and a fifth priority proportional to and decreasing with an age of its time stamp.

The personal server device may further be configured to transmit the parameter data to said one or more patient-specific medical devices in order according to the fifth priority; and delete the transmitted configuration data after receiving confirmation that the parameter data have been successfully transmitted; and delete non-transmitted data in order according to the fourth priority when storage capacity in the primary memory buffer reaches a predetermined limit.

Such a portable personal server device may be especially robust when connection is lost to the HCMS. In such a case, the PS will continue to receive measurement data from said at least one sensor but will not delete transmitted measurement data after receiving confirmation that the measurement data have been successfully transmitted because no such confirmation will be received.

Since the PS is portable and intended for continuous use in normal daily life of a patient, it is expected that the connection with the HCMS may be periodically unreliable, such as when the patient goes into a basement, a large building or travels through a tunnel. Accordingly, the data is stored in the primary memory buffer. In these cases the primary memory buffer will start filling up and something must be done to be able to continue receiving measurement data from said at least one sensor.

As the received measurement data may be important and relevant to the health of the patient, it should not be deleted without due consideration. It may be assumed that the most recent data is most important. Hence, if the primary memory buffer is running out of memory and thereby reaches a predetermined limit, the first priority is used to delete the oldest data first.

Further, in case the connection to the HCMS continues to be unsteady, the second priority is used to ensure that the most relevant and/or important data is sent as soon as possible.

The bandwidth of the connection to the HCMS may vary and in a normal state the bandwidth should be more than enough to send both the data being continually received from said one or more patient-specific medical devices and data having been temporarily stored in the primary memory buffer so that the buffer may be cleared once normal connections are established.

The price of the connection to the HCMS may also vary over time and the memory processing circuit may be configured to optimize when to transmit data that is not as relevant, important, and/or current to avoid high network costs.

The memory processing circuit may be a separate unit or part of the same unit of the PS.

The proportionality of the first priority to the age of the time stamp may be linear, logarithmical, exponential or any other suitable proportionality. An older age of the time stamp results in a higher first priority, i.e. data that are deleted before younger age data.

The proportionality of the second priority to the age of the time stamp may be linear, logarithmical, exponential or any other suitable proportionality and may be inversely proportional. An older age of the time stamp results in a lower second priority, i.e. data that are sent after younger age data.

The second wireless communication radio may be used to create a peer-to-peer connection between the personal server device and the health care management system such that they may communicate data directly with each other via the peer-to-peer connection and through firewalls; a dedicated connectivity server dynamically may mediate the direct peer-to-peer connection by providing respective IP-addresses to the personal server device and the health care management system; and the data may not be received by the connectivity server during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable PS, which may connect to different hubs using different connection types, e.g. switching from Wi-Fi to LTE as the user leaves their home.

The device may further be configured to decrypt encrypted data received by said one or more patient-specific medical devices and re-encrypt the data before transmitting the re-encrypted data to the health care management system.

This enables the PS to interpret the received data and further increases security of the received data before transmitting it, which may be sensitive and at risk. The re-encrypted data may be more securely encrypted than the received data and may conform to a standard encryption that the HCMS is able to decrypt efficiently.

The device may further be configured to extract features from the decrypted data of said one or more patient-specific medical devices, to encrypt the extracted features, and to transmit the encrypted extracted features to the health care management system.

Such features may be a sampling frequency or a heart rate extracted from the measurement data.

The first priority may be determined according to a first algorithm and the second priority is determined according to a second algorithm with a different proportionality to the age of the time stamp.

Considerations regarding deleting data may be different from considerations regarding transmitting data, accordingly different algorithms may be used to reflect this.

A different proportionality may be used to assign the first and second priorities depending on a type of the measurement data.

A type of the measurement data may be data coming from a specific sensor, having a specific size range, or having a specific format (e.g. waveform, image or heatmap).

By adapting the proportionality, certain types of data may have a relatively higher priority or increase/decrease in priority faster than other types of data. This may be relevant to reflect that a sensor or medical device or a set of medical devices providing a number of different measurement data may have different importance regarding the health of the patient. For example, for a patient with diabetes that is wearing a blood sugar sensor and also wearing a heart rate monitor, blood glucose data may be considered more important and therefore be deleted last and sent first compared to heart rate data.

The proportionality of a specific data type may be generic, for example an alarm may always have a very high priority to be sent first. The proportionality of a specific data type may be personalized, i.e. adapted to the patient, their needs, and/or a study being performed.

The memory processing circuit may be further configured to assign a third priority to the measurement data depending on a type of the measurement data; the personal server device may be configured to transmit the measurement data to the health care management system in order according to the second and/or the third priority; and the memory processing circuit may be configured to delete non-transmitted data in order according to the first and/or the third priority when storage capacity in the primary memory buffer reaches the predetermined storage limit.

A type of the measurement data may be data coming from a specific sensor, having a specific size range, or having a specific format (e.g. waveform, image or heatmap).

By using a third priority in addition to or instead of the first or second priority, more complicated concerns regarding which data to delete first or send first may be reflected. The third priority is not dependent on or proportional to the age of the data so may be better suited for certain types of data. Certain types of data may have an inherent relatively higher priority than others or increase/decrease in priority faster than other types of data. For example, an alarm may always have a high priority to be transmitted regardless of its age. As another example, some data may have a high priority to delete if it is a predetermined size but otherwise be treated as any other data with a priority proportional to age.

The algorithms used to determine the first, second and/or third priority may be personalized to the patient and/or their specific needs. This may include predetermined profiles for different types of patients or a custom set of algorithms and/or proportionalities for a specific patient.

This allows proportionality and/or values of specific data types to be personalized, i.e. adapted to the patient, their needs, and/or a study being performed on the patient.

The device may further comprise at least one secondary memory buffer with a lower read/write speed and/or a higher energy cost than the primary memory buffer. The memory processing circuit may be configured to: move measurement data from the primary memory buffer to the secondary memory buffer in order according to the first and/or third priority when storage capacity in the primary memory buffer reaches the predetermined storage limit; and delete data in the secondary memory buffer in order according to the first and/or the third priority when storage capacity in the secondary memory buffer reaches a predetermined secondary storage limit.

Accordingly, the primary memory buffer may be optimized for normal operations whereas said at least one secondary memory buffer may be optimized for temporary storage.

Any memory buffering functions of the primary memory buffer may be shared equally or unequally between the primary memory buffer and said at least one secondary memory buffer.

For example, the primary memory buffer may be of SRAM type and a secondary memory buffer may be of PSRAM type. In this example, the primary memory buffer has a higher read/write speed and uses less power than the secondary memory buffer, but a more limited memory space.

This may mean that the primary memory buffer is only incidentally used for temporary storage until there is a need for more substantial storage, such as whenever communications to the HCMS is interrupted. In such a case or similar ones, said at least one secondary memory buffer is used to temporarily store measurement data before communications are restored.

There may be more than one secondary memory buffer, such as a second secondary memory buffer and so on, each having a lower read/write speed and/or a higher energy cost than the previous one. In such a case, the memory processing circuit may be configured to move data from the secondary memory buffer to the second secondary memory buffer in a similar way that the data are moved from the primary memory buffer to the secondary memory buffer and so in. In such a case, data may only be deleted once all memory buffers are full, and in that case the data to be deleted may be determined by the first and/or third priority and further depend on which memory buffer the data is stored in.

For example, a memory buffer with limited write operations may only be used when necessary and only deleted when all other memories are close to empty. Such a write limit may also affect which priority ranges are stored in memory buffers with such a write limit below a predetermined threshold.

The device may further comprise at least one tertiary memory buffer less volatile than the primary memory buffer. The memory processing circuit may be configured to move measurement data from the primary memory buffer to the tertiary memory buffer in order according to the first and/or the third priority when a battery level of the portable personal server device reaches a predetermined charge.

For example, the primary memory buffer may be of SRAM type and a tertiary memory buffer may be of flash type. In this example, the primary memory buffer has a higher read/write speed and uses less power than the tertiary memory buffer, but a more limited memory space and flash is completely non-volatile unlike SRAM.

While the primary (and/or one or more secondary) memory buffer may have its own emergency battery supply, in cases where the data would not be guaranteed to be maintained after a (long) loss of battery power, moving at least the most important data to the tertiary memory buffer that is less or not at all volatile may be especially safe if the battery level of the PS reaches a critical point.

The device may be configured according to the eighth aspect of the invention.

The same alternatives and benefits apply mutatis mutandis.

According to an eleventh aspect of the invention, a method for managing limited temporary data storage in a portable device is provided. The method comprises steps of: continually receiving measurement data from at least one sensor, the measurement data comprising a time stamp corresponding to a time of measurement; assigning a first priority to the measurement data proportional to and increasing with an age of the time stamp; assigning a second priority to the measurement data proportional to and decreasing with the age of the time stamp; storing the measurement data in a primary memory buffer; transmitting measurement data for long-term storage in order according to the second priority; after receiving confirmation that the measurement data have been successfully transmitted, deleting the transmitted data; and when storage capacity in the primary memory buffer reaches a predetermined storage limit, deleting non-transmitted data in order according to the first priority.

Such a method may be implemented by and have similar alternatives and benefits as the tenth aspect of the invention.

Long-term storage may be considered as longer than 30 minutes, longer than 1 hour, longer than 24 hours, or longer than 2 days. Long-term storage may be any storage longer than what would be feasible for a portable unit not specialized for this purpose.

The proportionality of the first priority to the age of the time stamp may be linear, logarithmical, exponential or any other suitable proportionality. An older age of the time stamp results in a higher first priority, i.e. data that are deleted before younger age data.

The proportionality of the second priority to the age of the time stamp may be linear, logarithmical, exponential or any other suitable proportionality and may be inversely proportional. An older age of the time stamp results in a lower second priority, i.e. data that are sent after younger age data.

The first priority may be determined according to a first algorithm and the second priority is determined according to a second algorithm with a different proportionality to the age of the time stamp.

Considerations regarding deleting data may be different from considerations regarding transmitting data, accordingly different algorithms may be used to reflect this.

A different proportionality may be used to assign the first and second priorities depending on a type of the measurement data.

A type of the measurement data may be data coming from a specific sensor, having a specific size range, or having a specific format (e.g. waveform, image or heatmap).

By adapting the proportionality, certain types of data may have a relatively higher priority or increase/decrease in priority faster than other types of data. This may be relevant to reflect that a sensor or medical device or a set of medical devices providing a number of different measurement data may have different importance regarding the health of the patient. For example, for a patient with diabetes that is wearing a blood sugar sensor and also wearing a heart rate monitor, blood glucose data may be considered more important and therefore be deleted last and sent first compared to heart rate data.

The proportionality of a specific data type may be generic, for example an alarm may always have a very high priority to be sent first. The proportionality of a specific data type may be personalized, i.e. adapted to the patient, their needs, and/or a study being performed.

The method may further comprise steps of: assigning a third priority to the measurement data depending on a type of the measurement data; wherein transmitting measurement data for long-term storage is additionally or alternatively in order according to the third priority; and deleting non-transmitted data is additionally or alternatively in order according to the third priority.

A type of the measurement data may be data coming from a specific sensor, having a specific size range, or having a specific format (e.g. waveform, image or heatmap).

By using a third priority in addition to or instead of the first or second priority, more complicated concerns regarding which data to delete first or send first may be reflected. The third priority is not dependent on or proportional to the age of the data so may be better suited for certain types of data. Certain types of data may have an inherent relatively higher priority than others or increase/decrease in priority faster than other types of data. For example, an alarm may always have a high priority to be transmitted regardless of its age. As another example, some data may have a high priority to delete if it is a predetermined size but otherwise be treated as any other data with a priority proportional to age.

The algorithms used to determine the first, second and/or third priority may be personalized to the patient and/or their specific needs. This may include predetermined profiles for different types of patients or a custom set of algorithms and/or proportionalities for a specific patient.

This allows proportionality and/or values of specific data types to be personalized, i.e. adapted to the patient, their needs, and/or a study being performed on the patient.

The measurement data may be received, stored, and transmitted in an encrypted state.

This further increases security of the method.

The method may further comprise a step of encrypting the received measurement data; wherein the measurement data are stored and transmitted in an encrypted state.

This further increases the security of the method and enables secure transmission of data received from sensors that are not capable of encryption.

The method may further comprise steps of: moving measurement data from the primary memory buffer to a secondary memory buffer in order according to the first and/or third priority when storage capacity in the primary memory buffer reaches the predetermined storage limit; and deleting data in the secondary memory buffer in order according to the first and/or the third priority when storage capacity in the secondary memory buffer reaches a predetermined secondary storage limit.

Accordingly, the primary memory buffer may be optimized for normal operations whereas said at least one secondary memory buffer may be optimized for temporary storage.

Any memory buffering functions of the primary memory buffer may be shared equally or unequally between the primary memory buffer and said at least one secondary memory buffer.

This may mean that the primary memory buffer is only incidentally used for temporary storage until there is a need for more substantial storage, such as whenever communications to the HCMS is interrupted. In such a case or similar ones, said at least one secondary memory buffer is used to temporarily store measurement data before communications are restored.

There may be more than one secondary memory buffer, such as a quaternary and so on, each having a lower read/write speed and/or a higher energy cost than the previous one. In such a case, the memory processing circuit may be configured to move data from the secondary memory buffer to the quaternary memory buffer in a similar way that the data are moved from the primary memory buffer to the secondary memory buffer and so in. In such a case, data may only be deleted once all memory buffers are full, and in that case the data to be deleted may be determined by the first and/or third priority and further depend on which memory buffer the data is stored in. For example, a memory buffer with limited write operations may only be used when necessary and only deleted when all other memories are close to empty. Such a write limit may also affect which priority ranges are stored in memory buffers with such a write limit below a predetermined threshold.

The method may further comprise a step of moving measurement data from the primary memory buffer to a tertiary memory buffer in order according to the first and/or the third priority when a battery level of the portable personal server device reaches a predetermined charge.

While the primary (and/or one or more secondary) memory buffer may have its own emergency battery supply, in cases where the data would not be guaranteed to be maintained after a (long) loss of battery power, moving at least the most important data to the tertiary memory buffer that is less or not at all volatile may be especially safe if the battery level of the PS reaches a critical point.

The method may further comprise steps of reconstructing a timeline of measurement data based on the time stamps of the measurement data; and verifying whether any data are missing in the timeline.

This improves the functionality and readability of the data in a database and also enables simple detection whether data are missing.

Verifying whether any data are missing may comprise comparing a sampling frequency for the relevant data (which is controlled by the HCMS in some embodiments) with the reconstructed timeline and checking whether there are any missing data points where there should be data.

Verifying that data are missing may be relevant for the use of the data, for example some studies may require an uninterrupted set of data for a fixed time interval and if data are clearly marked as missing in a database it is easier to find usable data.

According to a twelfth aspect of the invention, a system for managing limited temporary data storage in a portable server device is provided. The system comprises: one or more patient-specific medical devices comprising at least one sensor, each medical device being configured to store measurement data received from said at least one sensor in a memory buffer and to encrypt the data into encrypted data; a health care management system configured to decrypt the encrypted data into decrypted data and to maintain a digital twin corresponding to a user of said one or more patient-specific medical devices comprising decrypted data of said one or more patient-specific medical devices; and a personal server device. The personal server device comprises: a first wireless communication radio for using a local wireless connection; a second wireless communication radio for using a secured and verified peer-to-peer connection; and a primary memory buffer. The personal server device is configured to: use a local wireless connection with said one or more patient-specific medical devices via the first wireless communication radio for transmitting encrypted data to and from said one or more patient-specific medical devices; use a secured and verified peer-to-peer connection with the health care management system via the second wireless communication radio for transmitting encrypted data to and from the health care management system; receive encrypted data from at least one sensor using the local wireless connection, the encrypted data comprising a time stamp corresponding to a time of measurement of the corresponding measurement data; assig a first priority to the encrypted data proportional to and increasing with an age of the time stamp; assig a second priority to the encrypted data proportional to and decreasing with the age of the time stamp; store the encrypted data in the primary memory buffer; transmit the encrypted data to the health care management system using the secured and verified peer-to-peer connection in order according to the second priority; delete the transmitted data after receiving confirmation that the encrypted data has been successfully transmitted; and delete non-transmitted data in order according to the first priority when storage capacity in the primary memory buffer reaches a predetermined storage limit.

Such a system may be implemented using and have similar alternatives and benefits as the tenth aspect of the invention.

The digital twin may be maintained by periodically updating the digital twin to comprise a copy of the measurement data and parameters as in said one or more patient-specific medical device.

This way the digital twin is ensured to be a match with said one or more patient-specific medical device such that any diagnoses or remedies applied using the digital twin also applies to said one or more patient-specific medical device.

The system may further comprise a dedicated connectivity server configured to dynamically mediate the peer-to-peer connection by providing respective IP-addresses to the personal server device and the health care management system; and to not receive data during the data transmission via the peer-to-peer connection.

Such a connectivity server enables a reliable and secure peer-to-peer connection. It is especially beneficial to be able to rely on a dedicated connectivity server to mediate IP-addresses in a secure manner for a portable PS, which may connect to different hubs using different connection types, e.g. switching from Wi-Fi to LTE as the user leaves their home.

The health care management system may be configured to reconstruct a timeline of decrypted data based on the time stamps of the received encrypted data and verify whether any data are missing in the timeline.

This improves the functionality and readability of the data in a database and also enables simple detection whether data are missing.

Verifying whether any data are missing may comprise comparing a sampling frequency for the relevant data (which is controlled by the HCMS in some embodiments) with the reconstructed timeline and checking whether there are any missing data points where there should be data.

Verifying that data are missing may be relevant for the use of the data, for example some studies may require an uninterrupted set of data for a fixed time interval and if data are clearly marked as missing in a database it is easier to find usable data.

The PS may be configured according to the eighth and/or tenth aspect of the invention.

The same alternatives and benefits apply mutatis mutandis.

### Description of drawings

The invention will be described in further detail with reference to preferred aspects and the accompanying drawing, in which:
Fig. 1 shows a schematic view of a system for bidirectional communication between a patient-specific medical device with at least one sensor and a health care management system according to an embodiment;
Fig. 2 shows a schematic view of a system for bidirectional communication between a patient-specific medical device with at least one sensor and a health care management system via a personal server device according to an embodiment;
Fig. 3 shows a health care management system according to an embodiment;
Fig. 4 shows a schematic view of a system for bidirectional communication between different patient-specific medical devices and a health care management system according to an embodiment;
Fig. 5 shows a schematic view of a system for bidirectional communication between different patient-specific medical devices and a health care management system according to an embodiment;
Fig. 6 shows a schematic view of a system for bidirectional communication between a patient-specific medical device and a health care management system according to an embodiment;
Fig. 7 shows a schematic view of a system for bidirectional communication between a patient-specific medical device and a health care management system via a personal server device according to an embodiment;
Fig. 8 shows a flowchart of a method for bidirectional communication between a patient-specific medical device with at least one sensor and a health care management system according to an embodiment;
Fig. 9 shows a flowchart of a method for maintaining a patient-specific digital twin of a health care management system according to an embodiment;
Fig. 10 shows a flowchart of a method for bidirectional communication between a patient-specific medical device with at least one sensor and a health care management system according to an embodiment;
Fig. 11 shows a flowchart of a method for bidirectional communication between a patient-specific medical device with at least one sensor and a health care management system via a personal server device according to an embodiment;
Fig. 12 shows a flowchart of a method for bidirectional communication between a patient-specific medical device with at least one sensor and a health care management system according to an embodiment;
Fig. 13 shows a flowchart of a method for bidirectional communication between a patient-specific medical device with at least one sensor and a health care management system via a personal server device according to an embodiment;
Figs. 14a-b show schematic views of a system for managing limited temporary data storage in a portable personal server device according to an embodiment;
Fig. 15 shows a schematic view of a system for managing limited temporary data storage in a portable personal server device according to an embodiment; and
Fig. 16 shows a flowchart of a method for managing limited temporary data storage in a portable device according to an embodiment.

### Description of embodiments

In the following and preceding, terms such as a/an/the and comprising are intended to be interpreted as non-limiting. The terms user and patient may be used interchangeably. Any processor or computing unit may be implemented as an electronic circuit and electronic connections may be wired or wireless unless otherwise explicitly stated. Unless otherwise context-dependent, the terms medical data, user data, measurement data, encrypted data, decrypted data, and signal may be used interchangeably. Unless explicitly specified, a wireless connection may be implemented in any number of standards known to a person skilled in the art, such as Wi-Fi, Bluetooth^{®}, Zigbee, Matter, 4G/LTE, 5G and so on.

The present invention presents systems and methods for secure and robust bidirectional communication between a patient and a server. There are a number of possible scenarios before the invention itself takes place, as e.g. first the system is set up and security credentials are created.

In one such scenario, the patient has been diagnosed with a condition that requires follow-up and monitoring. In another, the patient is suspected to have condition and further data may be collected for a more accurate diagnosis.

As an example, a medical professional sets up a clinical study for the patient. The patient receives one or more medical devices 10 (MD) with at least one sensor 12 to use/wear during their day-to-day life. The medical professional wants to access data from the MD(s) 10 to better diagnose the patient, and there are many benefits to collect this data in real-time and remotely. The medical professional or a programmer therefore sets up a digital twin 22 corresponding to the patient and sets up security credentials for the MD(s) 10 and a health care management system (HCMS) 20 comprising the digital twin 22. The digital twin 22 also comprises a digital twin of the MD 10 that may also be set up when the digital twin 22 is set up.

Alternatively, the digital twin 22 may be set up in advance but be waiting for activation until the MD 10 is set up/activated, such as after the MD 10 is purchased at a pharmacy or general store and activated by a user 1 at home. The MD 10 then connects to a previously authorized HCMS 20, which activates a digital twin 22 corresponding to the user 1 and comprising a twin of the MD 10.

When setting up the digital twin 22 corresponding to the patient, this may comprise creating the patient in a picture archive system (PACS) and assigning MDs 10 to it in that system. Each hospital has their own PACS system, it is a medical standard and there are other software implementations using PACS. MDs 10 may have a barcode or security key that is verified when it is assigned.

In a scenario where the medical device provider sets up its own digital twin 22 to be connected to an MD 10 sold by the medical device provider, the medical device provider may have their own system different from PACS.

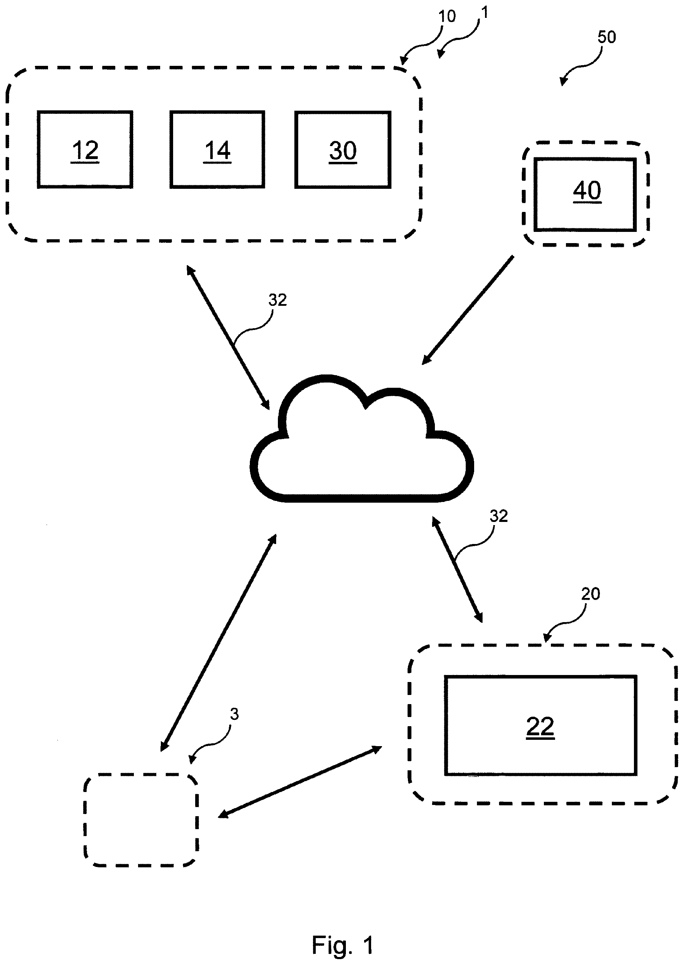

Fig. 1 shows an embodiment of a system 50 for bidirectional communication between a patient-specific medical device (MD) 10 with at least one sensor 12 and a health care management system (HCMS).

The MD 10 is configured to store measurement data received from said at least one sensor 12 in a memory buffer 14 and encrypt the data into encrypted data.

The HCMS 20 is configured to decrypt the encrypted data into decrypted data and maintain a digital twin 22 corresponding to a user 1 of the MD 10 comprising decrypted data of the MD 10 (corresponding to measurement data and potentially other data). The decrypted data is stored in the digital twin 22 as they are received and for as long as they are relevant or until a memory limit is reached.

The system 50 further comprises a network module 30. In Fig. 1, the network module 30 is part of the MD 10. The network module 30 is configured to establish or use a secured and verified peer-to-peer connection 32 between the MD 10 and the HCMS 20.

The MD 10 is further configured to transmit the encrypted data to the HCMS 20 via the secured and verified peer-to-peer connection 32 in real-time or during a scheduled/periodic update.

The system 50 further comprises a dedicated connectivity server 40 configured to dynamically mediate the peer-to-peer connection 32 by providing respective IP-addresses to the MD 10 and the HCMS 20. The connectivity server 40 is further configured to not receive (any) data during the data transmission via the peer-to-peer connection 32.

The connectivity server 40 thereby only mediates the peer-to-peer connection 32 and does not itself form part of the peer-to-peer connection 32. The security of the peer-to-peer connection 32 is thereby maintained, as no sensitive data are received by the connectivity server 40.

While the peer-to-peer connection 32 is illustrated as two separate arrows in Fig. 1, in reality the peer-to-peer connection 32 will likely be dozens or hundreds of data transmissions between different IP hubs, servers, and base stations, sometimes split into several different parallel channels or frequencies. However, the intermediaries of the transmission will not and possibly cannot access or read the data being transmitted.

The dedicated connectivity server 40 ensures that the peer-to-peer connection 32 is still peer-to-peer even if e.g. the MD 10 is in motion by providing the respective IP-addresses as they change (i.e. dynamically). This way, all data will be transmitted to the correct IP-address.

This dynamic ability of the dedicated connectivity server 40 to update either the MD 10 or the HCMS 20 is shown in Fig. 1 as bidirectional arrows, not to be misconstrued as an alternative channel for communication between the MD 10 and the HCMS 20.

The dedicated connectivity server 40 may further enable and/or enhance the security of the secured and verified peer-to-peer connection.

A virtual channel 36 may be assigned for each data or group of data being transmitted via the secured and verified peer-to-peer connection.

Such virtual channels may be used to more efficiently transmit different types of data, such as data coming from a specific sensor, having a specific size range, or having a specific type (e.g. waveform or heatmap).

Each virtual channel 36 may have a relative priority. By each virtual channel having a relative priority, data that is more time-sensitive (such as event data triggering a notification to a third party or the notification itself) may be transmitted faster.

The system 50 may further comprise a third party 3. The third party 3 may be a relative, caretaker, medical professional, or any other relevant third party. The third party 3 may have access to the HCMS 20, e.g. by credentials set up when setting up the digital twin 22. Some third parties 3 may further have previously established general access, e.g. a medical device provider or a medical professional.

The user 1 and/or the third party 3 may access the HCMS 20 to receive analysis, access data, control MD(s) 10 or any other suitable function making use of the digital twin 22. Such functions may be available in real-time and use up-to-date (historical/decrypted) data from the MD(s).

Contact details of the third party 3 may be comprised in the digital twin 22. On occasion, such as when detecting an event that may require action, the third party 3 may be contacted by the MD 10 or the HCMS 20 using any number of methods such as cellular (text message or phone call) or IP (email or pop-up notification).

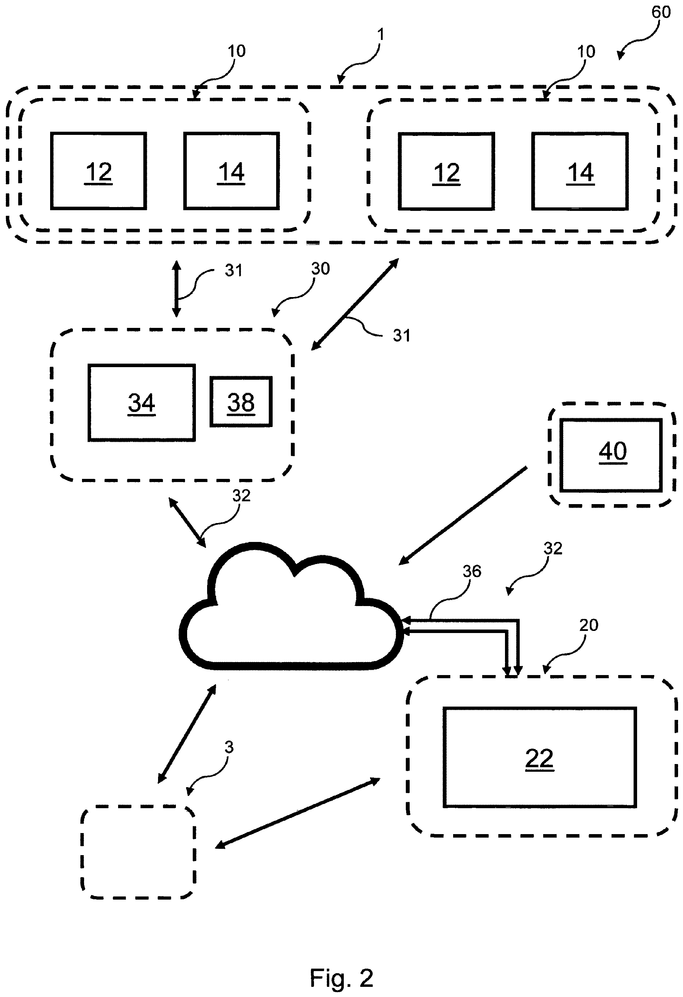

Fig. 2 is a similar system for bidirectional communication between an MD 10 with at least one sensor 12 and an HCMS 20 as in Fig. 1, but the system 60 in Fig. 2 further comprises a personal server device (PS) 30. The PS 30 may fulfil a similar role as the network module 30 in Fig. 1.

The PS 30 may be portable or stationary. The PS 30 may enable communication with an HCMS 20 for MD(s) 10 without an internal network module capable of long-distance communication. Even for MD(s) 10 with such capabilities, and for those without, the PS 30 may offer further capabilities, such as additional sensors, an extended memory buffer 34, a larger battery capacity, a better processor, and larger and different antennas. The PS 30 may also act as a common hub for communications between several MDs 10 and the HCMS 20.

The PS 30 comprises a first wireless communication radio for establishing or using a first wireless connection 31, a second wireless communication radio for establishing or using a second wireless connection 32, one or more (micro)processors, and a memory buffer 34.

The PS 30 is configured to establish or use a local wireless connection 31 with one or more MD(s) 10 via the first wireless communication radio for transmitting encrypted data to and from the MD(s) 10, and establish or use a secured and verified peer-to-peer connection 32 with the HCMS 20 via the second wireless communication radio for transmitting encrypted data to and from the HCMS 20.

Using the first and second wireless connections 31, 32, the PS 30 is further configured to transmit encrypted data from the MD(s) 10 to the HCMS 20 and from the HCMS 20 to MD(s) 10, execute error detection on the first and second wireless connections 31, 32, and buffer the encrypted data received by the MD(s) 10 to be transmitted to the HCMS 20 until the encrypted data are safely received by the HCMS 20.

A dedicated connectivity server 40 may mediate the peer-to-peer connection 32 between the PS 30 and the HCMS 20 in a similar manner as the dedicated connectivity server 40 in Fig. 1 mutatis mutandis.

This dynamic ability of the dedicated connectivity server 40 to update either the PS 30 or the HCMS 20 is shown in Fig. 2 as bidirectional arrows, not to be misconstrued as an alternative channel for communication between the PS 30 and the HCMS 20.

The dedicated connectivity server 40 may further enable and/or enhance the security of the secured and verified peer-to-peer connection.

When the PS 30 acts as a common hub for communications between several MDs 10 and the HCMS 20, the different data from the different MDs 10 may be handled in several different ways.

For example, the different data from the different MDs 10 may be collected in a shared memory buffer 34 and compared against each other to find correlations and secondary indicators, which may be considered as extracted features. These extracted features may trigger action such as changing operational parameters of at least one of the MDs 10 and may be sent to the HCMS 20 to be added to the digital twin 22.

As another example, the PS 30 may detect specific aspects of the data from the different MDs 10 that may trigger specific actions. For example, if data contains an event flag or other data indicating that a medical event is occurring potentially putting the user 1 at risk, this may cause the PS 30 to send an alarm/notification to emergency services or other suitable third parties 3, or cause a specific MD 1 to take action, such as executing a controlled injection or changing the voltage or intensity of a pacemaker.

If there is more than one MD 10, as there are in the example of Fig. 2, the PS 30 may use the same or similar first wireless connection 31 to communicate with each of the different MDs 10.

If there are more than one HCMS 20, the PS 30 will use a different peer-to-peer connection 32 with each of the HCMSs 20.

Each HCMS 20 may be associated with a user 1, an MD 10, a sensor 12 of an MD 10, a PS 30, or a combination of these.

If there are more than one MD 10 and one HCMS 20, as there are in the example of Fig. 2, the PS 30 may use the same or a different peer-to-peer connection 32 depending on the embodiment.

When using the same peer-to-peer connection 32, each MD 10 may or may not use at least one separate virtual channel 36 of the peer-to-peer connection 32 to separate data from different MDs 10.

When using a different peer-to-peer connection 32 for each MD 10, each peer-to-peer connection 32 may still have virtual channels 36, that e.g. separate data with different priority or content. Such a separation of data may also be applied when each MD 10 has at least one separate virtual channel 36.

The PS 30 will receive encrypted data from the MDs 10 using the first wireless connection 31 because even at close range, the security of medical data is imperative to maintain.

The PS 30 may decrypt the data it receives, either from the MDs 10 using the first wireless connection 31, from the HCMS 20 using the second wireless connection 32, or both. This enables the PS 30 to further analyse and extract features from the data.

If the PS 30 decrypts data, it also re-encrypts the data. This re-encryption may be the same or different from the original encryption.

It may be beneficial that the encryptions are different, as different methods of communication may be more suitable to different encryptions. If the encryptions are different, this enables the HCMS 20 to have an encryption that is only known by the PS 30 and not by the MDs 10, further increasing security.

The PS 30 may further comprise different sensors and functions that assist the MDs 10 and/or the HCMS 20.

For example, the memory buffer 34 of the PS 30 may be much bigger than memory buffers 14 of the MDs 10, enabling more data to be stored before being transmitted to the HCMS 20. This may be beneficial if connection is lost or unstable.

As another example, the PS 30 may periodically synchronize internal clocks of the MDs 10. This ensures the MDs' 10 time stamps don't drift in relation to each other over time. This may use an external clock that is more precise than the MDs 10 would otherwise have access to, such as GPS time.

As another example, the PS 30 may provide position data to the MDs 10 and/or the HCMS 20.

The PS 30 in Fig. 2 further comprises a button 38. This button 38 may have any number of functions and they may depend on the MDs 10 connected to the PS 30. The button 38 may e.g. turn on or off all the MDs 10 and the PS 30, trigger an alarm/event, or trigger a measurement to be made.

The memory buffer 34 of the PS 30 may be used to store data received by the MDs 10 until they are transmitted to the HCMS 20. This may comprise storing the data until the transmission is verified by the HCMS as having been completely and securely received.

The PS 30 executes error detection on the first and second wireless connections 31, 32. Error detection may comprise detecting whether received data is corrupted and/or incomplete, which may comprise any number of techniques for error detection known in the art, such as parity bits, or cyclic redundancy check (CRC) codes.

Sometimes errors may be corrected automatically, and sometimes retransmission may be required because the data are too corrupt to be corrected automatically. The autocorrection may use redundancy in the transmission to correct what may be missing. For example, Bluetooth may use frequency hopping that distributes data across different streams so that even if one stream is lost all data may be recovered using the other streams.

Either end of receiving a transmission, such as the PS 30 or the HCMS 20, may apply a logical scheme to determine whether the data are correctly received and reply with a confirmation receipt.

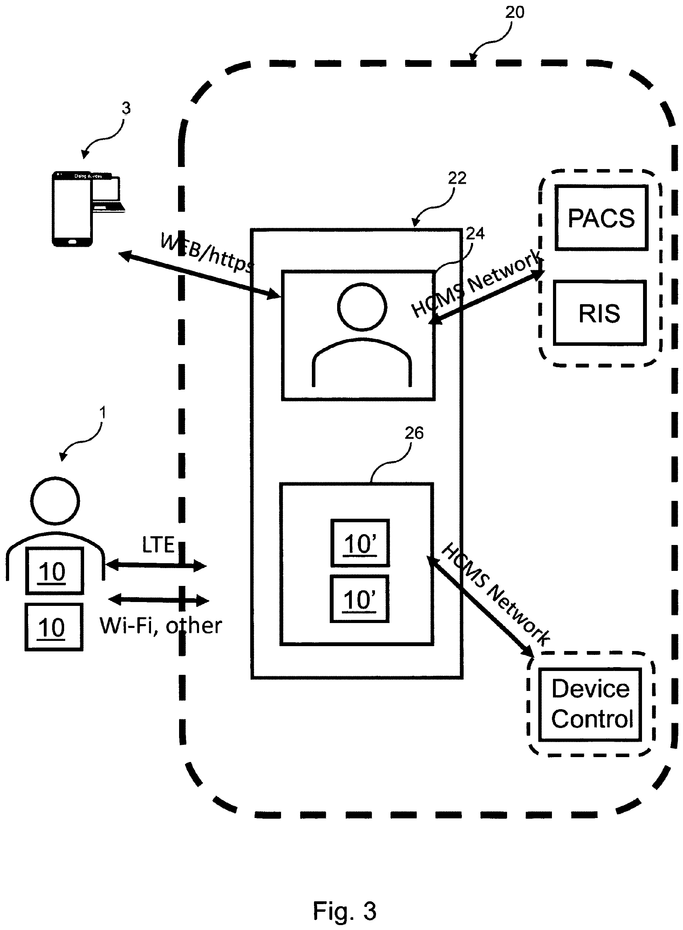

Fig. 3 shows an example of an HCMS 20. The HCMS 20 comprises a digital twin 22, which in turn has a patient-specific memory 24 and a device-specific memory 26.

The digital twin 22 is patient-specific in that it belongs to a user 1 and contains data of that user 1. A user 1 may have more than one digital twin 22, but a digital twin 22 may not have more than one user 1.

A digital twin 22 is a virtual representation of a physical object or system, creating a real-time digital counterpart. It integrates data from sensors and other sources to mirror the physical entity's behavior and characteristics. Examples include manufacturing, where a digital twin of a product streamlines design and production processes and in healthcare, patient-specific digital twins aid in personalized treatment planning.

In Fig. 3, the digital twin 22 is a part of the back-end software residing in the HCMS. The systems 50, 60 according to Figs. 1 and 2 ensure the wireless data transport between the digital twin 22 and the MDs 10, which might be interrupted from time to time for physical reasons. The digital twin 22 holds up-to-date information about the patient 1, the parameters for and the state of the MDs 10 and all measured data from the MDs 10, all in DICOM format, and the systems 50, 60 update the data whenever a connection is available.

The HCMS 20 is configured to continuously receive measurement data from sensors 12 of MDs 10 belonging to the user 1. The medical data are stored in the patient-specific memory 24, data such as ECG, EEG, audiogram, or blood analysis. These medical data correspond to the measurements done by the sensors 12 of MDs 10 belonging to the user 1. The medical data may be stored in a standardized manner.

The HCMS 20 is configured to continuously synchronize the data in the device-specific memory 26 with the corresponding MDs 10. This synchronization may be in real-time and bidirectional, any change to the MDs 10 is stored in the device-specific memory 26 and any change to the device-specific memory 26 is stored in the MDs 10.

There are many scenarios for when a user 1 may have several MDs 10. The user 1 may have several different sensors 12 measuring data, the user 1 may have MDs 10 that cooperate with each other such as hearing aids, or the user 1 may have use of a single MD type that has two separate units, e.g. one for use during daytime and one for use during night-time.

Each MD 10 has a unique twin MD in the digital twin 22. In the case of cooperating MDs 10, they may still be treated as different MDs in the digital twin 22.

In the case of one MD type with different units, each unit may or may not have a different twin MD, depending on whether the parameters of the MD 10 should be kept constant when alternating between the different units. If the parameters are to be kept constant, it may be beneficial for the different units to share a twin MD. This may be especially beneficial e.g. when replacing an MD, such that the same parameters are automatically transferred to the new MD.

Regardless of the number of MDs 10, all measurement data are stored in the patient-specific memory 24. There may be a record of how the measurement was taken and which MD 10/sensor 12 was used, which may be stored in either memory 24, 26 or as metadata.

As long as the measurement data and parameter data have a time-stamp and the measurement data have a way to identify the MD 10/sensor 12 used, the conditions of the measurement may be fully reconstructed by cross-referencing measurement data stored in the patient-specific memory 24 with parameter data stored in the device-specific memory 26.

The digital twin 22 may further store other types of metadata, such as age, sex, insurance information and contact information.

The HCMS 20 may further comprise or interface with other software, such as internal databases of a hospital or a website.

For example, a website may be granted access to the patient-specific memory 24 and use the data there to create a summary of the condition of the patient. This summary may be accessed by authorized third parties, such as a relative or caretaker of the patient, by logging into the website. The website may further monitor the data for indications of a medical event or emergency and act in response to this.

The patient-specific memory 24 may further be accessed by the patient's local hospital, which uses the data there to create a local medical record file in their picture archive system (PACS) and/or radiology information system (RIS).

The HCMS 20 may further use hospital or external trusted resources to control and/or optimize the MDs 10 via the device-specific memory 26.

The MDs 10 may be controlled and/or optimized by changing the twin MDs 10' in the device-specific memory 26, as the device-specific memory 26 is continuously synchronized to the actual MDs 10.

In one embodiment, the MDs 10 are only controllable by affecting the digital twin 22, e.g. through controlling a corresponding twin MD 10'. This may mean that the MD 10 is configured to resist other control data such that the only way to control the MD 10 is through controlling a corresponding twin MD 10'. This may further enhance security, as the corresponding twin MD 10' is easier to secure.

The MDs 10 may be optimized by using data in the device-specific memory 26 and/or the patient-specific memory 24. This may comprise referencing external databases and using neural networks or similar big data/AI structures.

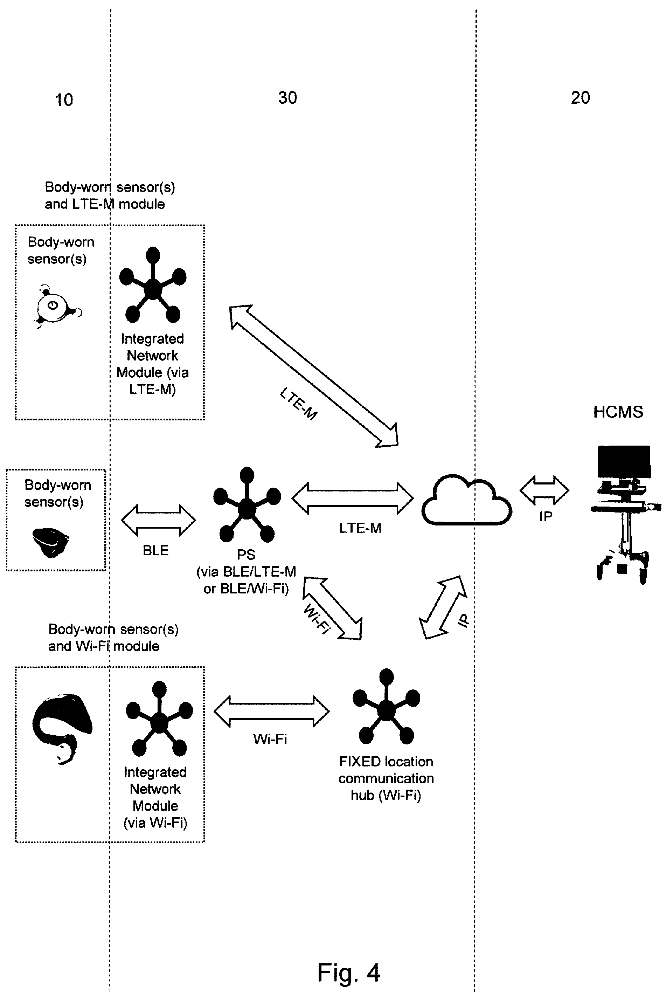

Fig. 4 shows different connections between different MDs 10, an HCMS 20 and different network modules 30.

At the top of the different MDs 10 is an MD 10 with an integrated LTE-M module 30. This MD 10 can communicate directly with the internet and the HCMS 20 with the integrated LTE-M module 30.

In the middle of the different MDs 10 as a simple MD that only comprises sensor(s). The MD 10 uses BLE to communicate with a PS 30. The PS 30 in turn communicates either directly with the internet and the HCMS 20 with its own LTE-M module or with a local router using a Wi-Fi module. The local router then communicates with the internet and the HCMS 20.

At the bottom of the different MDs 10 is an MD 10 with an integrated Wi-Fi module 30. This MD 10 can communicate with the local router using Wi-Fi. The local router then communicates with the internet and the HCMS 20.

Despite all these different connections using different methods, components, protocols and intermediaries; they are all compatible with secured and verified peer-to-peer connection between the different MDs 10, PS 30, and the HCMS 20.

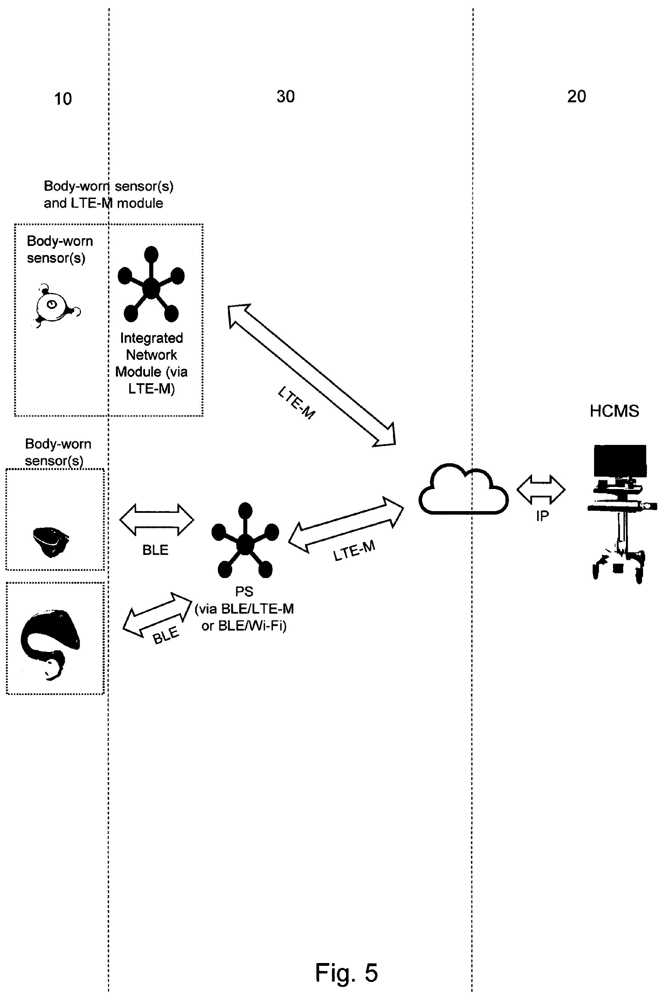

Fig. 5 shows different connections between different MDs 10, an HCMS 20 and different network modules 30.

The top MD 10 is the same top MD 10 as in Fig. 4.

The other MDs 10 in Fig. 5 use BLE to communicate with the same PS 30. The PS 30 in turn communicates directly with the internet and the HCMS 20 with its own LTE-M module. In another embodiment not shown, the PS 30 may communicate with a local router using a Wi-Fi module as in Fig. 4.

Despite the PS 30 communicating with several different MDs 10, the PS 30 still facilitates a secured and verified peer-to-peer connection between each of the different MDs 10 and the HCMS 20.

Each of the MDs 10 may use the same or a different secured and verified peer-to-peer connection between the PS 30 and the HCMS 20. This secured and verified peer-to-peer connection is then extended to each of the MDs 10 by the BLE communication between the PS 30 and the MDs 10.

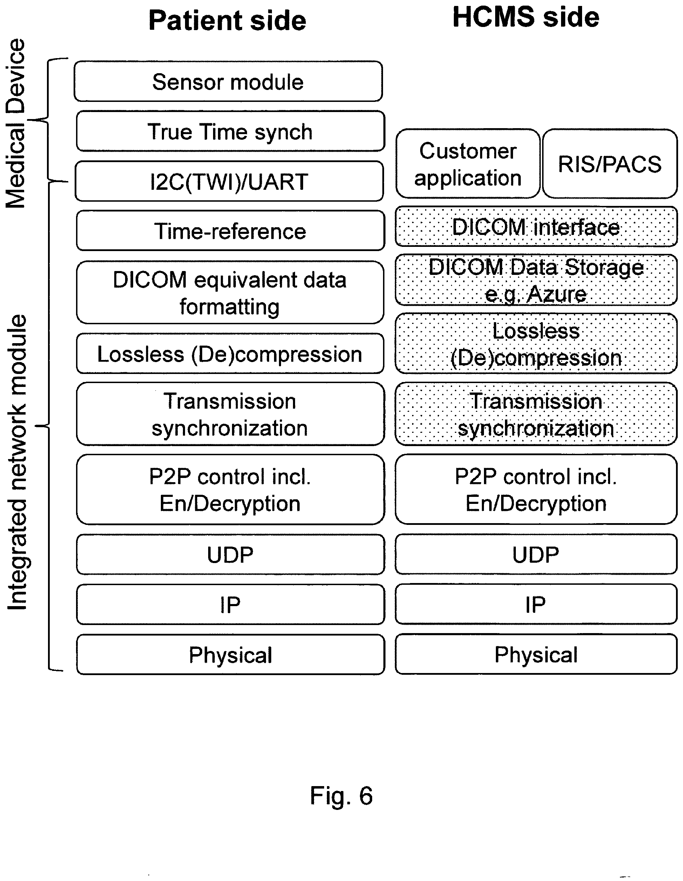

Fig. 6 shows a specific example embodiment of different software- and hardware modules that enable the system as in Fig. 1.

On the patient side, starting from the top there is a medical device comprising a sensor module configured to sense (medical) measurement data.

Next in the flow, an internal clock of the medical device is synchronized to true time, which may e.g. be GPS precise time, to enable using an absolute time stamp. This step may be independent of the measurement data being sensed and transmitted, and may occur at regular intervals.

Next, the medical device interfaces with an integrated network module, using a 12C(TWI)/UART hardware connection.

Next, the time-reference step is similar to the true time synch in that it may be independent of the measurement data being sensed and transmitted, and may occur at regular intervals. In the time-reference step, the system time of the PS is updated against an independent source, such as network time or GPS time.

Next, the data are formatted to a standard (if not already correctly formatted), in this case DICOM. When formatting for DICOM, time stamp info (already present e.g. as metadata from measurement) may be changed into a start and a stop time for a block of data with fixed delay between the samples. At this point, the data exist as current data in a memory buffer.

Next, the data are compressed losslessly. When receiving data from the HCMS, this is instead where data are decompressed.

Next, a transmission synchronization module manages the transmission and the memory buffer. The transmission synchronization module controls when to transmit new data, to ensure that enough new data has been measured to fill a packet and be possible to encrypt. The transmission synchronization module also manages the memory buffer such that it deletes data that have been received by the HCMS and sends and receives verification when data are received correctly.

Next, the data are encrypted and a secured and verified peer-to-peer connection is established/managed between the network module and the HCMS.

Next, the data are packaged according to UDP protocols.

Next, the data are prepared to be sent over the internet to the HCMS.

Finally, a physical layer comprising radio hardware such as circuits and antennas. This is where the raw bit streams are transmitted over a physical medium to reach the other end.

On the HCMS side, the data are received bottom to top. Starting from the bottom, the physical layer enables receiving the raw bit stream using radio hardware.

Next, the data are received over the internet using the secured and verified peer-to-peer connection.

Next, the packages are unpacked and checked for errors or missing data.

Next, the data are decrypted using security means previously established between the medical device and the HCMS.

Next, the transmission synchronization module manages the transmission in a similar manner as on the patient side. When receiving data, the transmission synchronization module sends verification when data are received correctly.

Next, the data are decompressed (losslessly). When transmitting data from the HCMS, this is instead where data are compressed.

Next, the data are stored in the digital twin using standardized means, in this case Azure for DICOM.

Next, the data are available for analysis, interpretation, and view.

These last four steps, marked with a pattern in Fig. 6, correspond to maintaining the digital twin.

Next, a patient, medical professional or third party may access at least parts of the data using e.g. a customer application (patient-adapted data presentation) or RIS/PACS (medical-professional-adapted data presentation and manipulation/analysis).

Note that data may also flow in the opposite direction, from the top of the HCMS side, through the bottom of both columns, to the top of the patient side.

This is only one example embodiment, some steps may be skipped, added, moved or performed simultaneously in other embodiments.

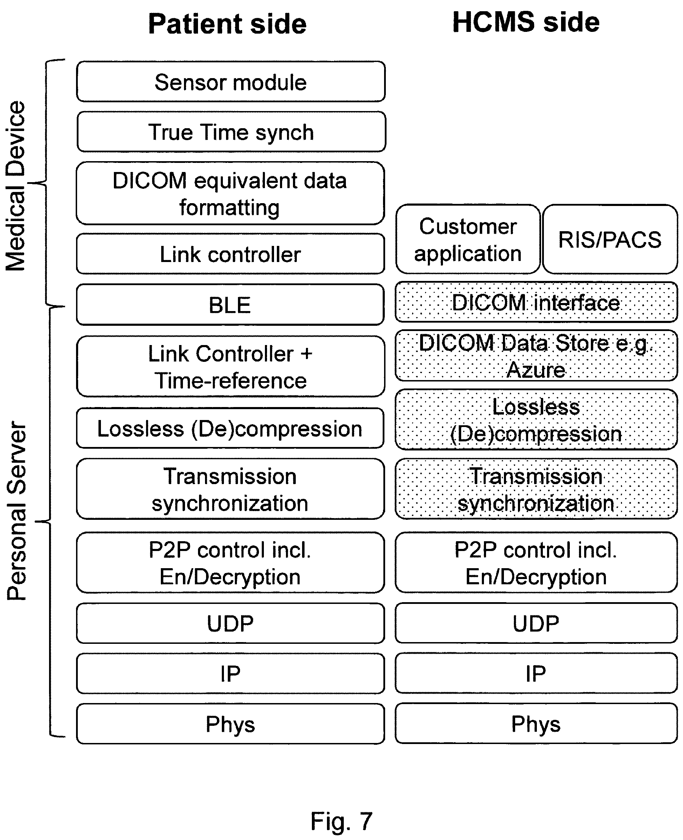

Fig. 7 shows a specific example embodiment of different software- and hardware modules that enable the system as in Fig. 2.

On the patient side, starting from the top there is a medical device (MD) comprising a sensor module configured to sense (medical) measurement data.

Next in the flow, an internal clock of the medical device is synchronized to true time, which may e.g. be system time received by a personal server device (PS), to enable using an absolute time stamp. This step may be independent of the measurement data being sensed and transmitted, and may occur at regular intervals.

Next, the data are formatted to a standard (if not already correctly formatted), in this case DICOM. When formatting for DICOM, time stamp info (already present e.g. as metadata from measurement) may be changed into a start and a stop time for a block of data with fixed delay between the samples.

Next, a link controller controls the data sent over BLE. This comprises managing pairing of the MD and PS and encrypting/decrypting the signal on either end of the transmission. The encryption is a standard BLE encryption.

Next, the medical device interfaces with the PS using BLE.

Next, link controller encrypts/decrypts the BLE signal and manages pairing in a corresponding manner. At this point, the data exist as current data in a memory buffer of the PS.

The time-reference step is similar to the true time synch in that it may be independent of the measurement data being sensed and transmitted, and may occur at regular intervals. In the time-reference step, the system time of the PS is updated against an independent source, such as network time or GPS time.

Next, the data are compressed losslessly. When receiving data from the HCMS, this is instead where data are decompressed.

Next, a transmission synchronization module manages the transmission and the memory buffer. The transmission synchronization module controls when to transmit new data, to ensure that enough new data has been measured to fill a packet and be possible to encrypt. The transmission synchronization module also manages the memory buffer such that it deletes data that have been received by the HCMS and sends and receives verification when data are received correctly.

Next, the data are encrypted and a secured and verified peer-to-peer connection is established between the PS and the HCMS.

Next, the data are packaged according to UDP protocols.

Next, the data are prepared to be sent over the internet to the HCMS.

Finally, a physical layer comprising radio hardware such as circuits and antennas. This is where the raw bit streams are transmitted over a physical medium to reach the other end.

On the HCMS side, the data are received bottom to top. Starting from the bottom, the physical layer enables receiving the raw bit stream using radio hardware.

Next, the data are received over the internet using the secured and verified peer-to-peer connection.

Next, the packages are unpacked and checked for errors or missing data.

Next, the data are decrypted using security means previously established between the medical device and the HCMS.

Next, the transmission synchronization module manages the transmission in a similar manner as on the patient side. When receiving data, the transmission synchronization module sends verification when data are received correctly.

Next, the data are decompressed (losslessly). When transmitting data from the HCMS, this is instead where data are compressed.

Next, the data are stored in the digital twin using standardized means, in this case Azure for DICOM.

Next, the data are available for analysis, interpretation, and view.

These last four steps, marked with a pattern in Fig. 7, correspond to maintaining the digital twin.

Next, a patient, medical professional or third party may access at least parts of the data using e.g. a customer application (patient-adapted data presentation) or RIS/PACS (medical-professional-adapted data presentation and manipulation/analysis).

Note that the HCMS side is identical in Figs. 6 and 7. The HCMS is not affected by how the data are sent.

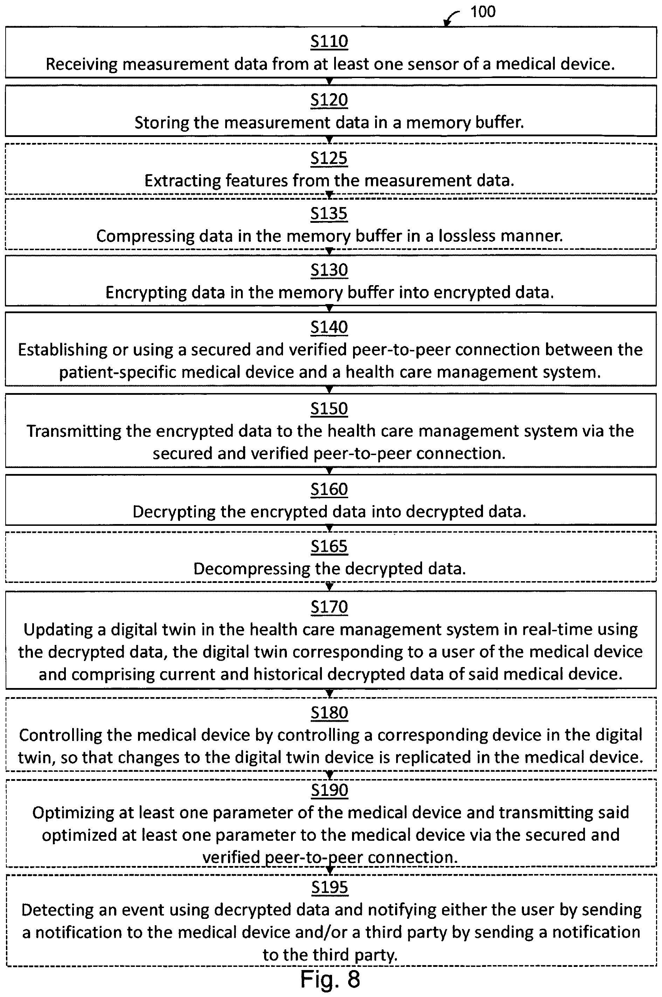

Fig. 8 shows a method 100 for bidirectional communication between a patient-specific medical device with at least one sensor and a health care management system (HCMS).

The method 100 comprises a number of steps, some of which are optional. The optional steps are marked in Fig. 8 with dashed boxes. Steps may be performed in a different order or simultaneously.

The first step is receiving S110 measurement data from at least one sensor of a medical device. This step S110 may be performed e.g. by a processing circuit or memory buffer of the medical device. This step S110 may further be performed by a separate device from the medical device, such as a personal server device.

The next step is storing S120 the measurement data in a memory buffer. This step S120 may be performed by the medical device or by a separate device, such as a personal server device.

Storing S120 may further comprise storing parameters of the medical device at a time of measuring.

Storing S120 may further comprise storing an absolute time stamp for each measurement or set of measurements corresponding to the measurement data in the memory buffer.

The next step is extracting S125 features from the measurement data. This step S125 is optional and may be performed e.g. by a processing circuit on some or all of the data in the memory buffer.

Storing S120 may further comprise storing the extracted features in the memory buffer.

The next step is compressing S135 data in the memory buffer in a lossless manner. This step S135 is optional and may be performed e.g. by a processing circuit on some or all of the data in the memory buffer.

The next step is encrypting S130 data in the memory buffer into encrypted data. This step S130 may be performed e.g. by a processing circuit on some or all of the data in the memory buffer.

The steps of compressing S135 and encrypting S130 may be performed in one combined operation.

The next step is establishing or using S140 a secured and verified peer-to-peer connection between the patient-specific medical device and the health care management system. This step S140 may be performed by the medical device, a network module (of the medical device or another device), and/or a connectivity server.

Establishing S140 a secured and verified peer-to-peer connection may comprise establishing a bidirectional system for verifying integrity of data being transmitted using the connection.

Establishing S140 a secured and verified peer-to-peer connection may comprise using a dedicated connectivity server to dynamically mediate the direct peer-to-peer connection by providing respective IP-addresses to the patient-specific medical device and the health care management system.

The next step is transmitting S150 the encrypted data to the health care management system via the secured and verified peer-to-peer connection. This step S150 may be performed by the medical device or a network module (of the medical device or another device).

The next step is decrypting S160 the encrypted data into decrypted data. This step S160 is performed by the HCMS.

The next step is decompressing S165 the decrypted data. This step S165 is optional and performed by the HCMS. This step S165 is usually performed if the optional step S135 of compressing data was performed.

The next step is updating S170 a digital twin in the health care management system in real-time using the decrypted data, the digital twin corresponding to a user of the medical device and comprising decrypted data of said medical device. This step S170 is performed by the HCMS.

Updating S170 the digital twin may comprise maintaining a copy of the measurement data and parameters in the medical device and the digital twin.

The next step is controlling S180 the medical device by controlling a corresponding device in the digital twin, so that changes to the digital twin device is replicated in the medical device. This step S180 is optional and performed by the HCMS. In this case, the medical device may be configured to resist certain control data, e.g. control data not received using this method step S180.

The next step is optimizing S190 at least one parameter of the medical device and transmitting said optimized at least one parameter to the medical device via the secured and verified peer-to-peer connection. This step S190 is optional and performed by the HCMS.

The optimizing step S190 may comprise optimizing at least one parameter of the medical device using decrypted data of said at least one sensor comprised in the medical device and/or measurement data of at least one sensor not comprised in the medical device and comprised in the digital twin.

The optimizing step S190 may further comprise using a database of algorithms and/or parameters to optimize said at least one parameter.

The next step is detecting S195 an event using decrypted data and notifying either the user by sending a notification/alarm to the medical device and/or a third party by sending a notification/alarm to the third party. This step S195 is optional and performed by the HCMS.

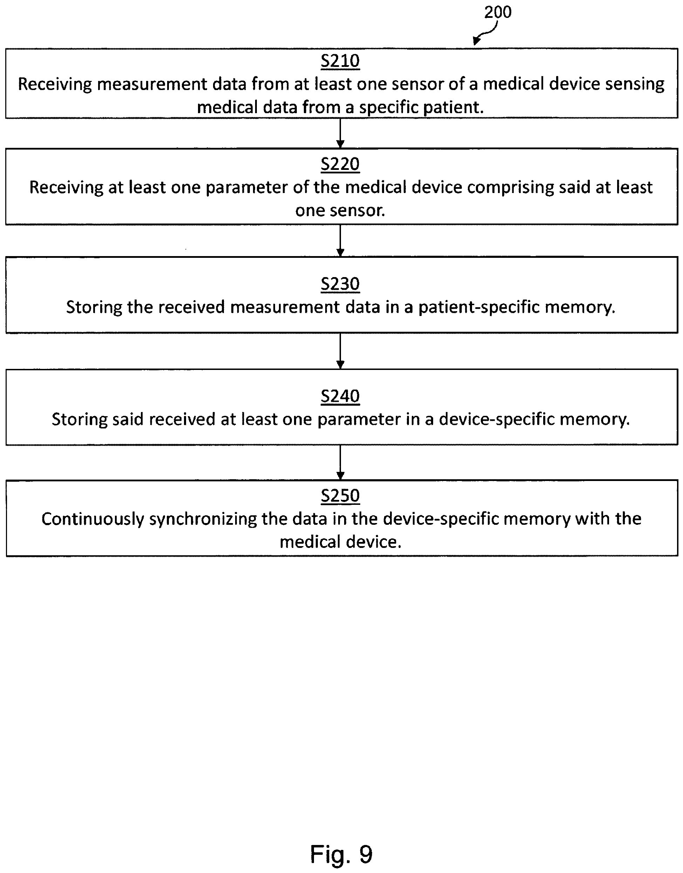

Fig. 9 shows a method 200 for maintaining a patient-specific digital twin.

The method 200 comprises a number of steps, which may be performed in a different order or simultaneously.

The first step is receiving S210 measurement data from at least one sensor of a medical device sensing medical data from a specific patient. This step S210 is performed by a health care management system (HCMS). The data may be received using a secured and verified peer-to-peer connection to the medical device or network module/personal server device/router connected to the medical device.

The next step is receiving S220 at least one parameter of the medical device comprising said at least one sensor. This step S210 is performed by the HCMS. The data may be received using a secured and verified peer-to-peer connection to the medical device, or to a network module/personal server device/router connected to the medical device.

The next step is storing S230 the received measurement data in a patient-specific memory (of the digital twin). This step is performed by the HCMS.

The next step is storing S240 said received at least one parameter in a device-specific memory (of the digital twin). This step is performed by the HCMS.

The next step is continuously synchronizing S250 the data in the device-specific memory with the medical device. This step is performed by the HCMS. This step is performed continuously, and may be performed simultaneously with other steps of the method 200. Synchronizing S250 the data may use a secured and verified peer-to-peer connection to the medical device, or to a network module/personal server device/router connected to the medical device.

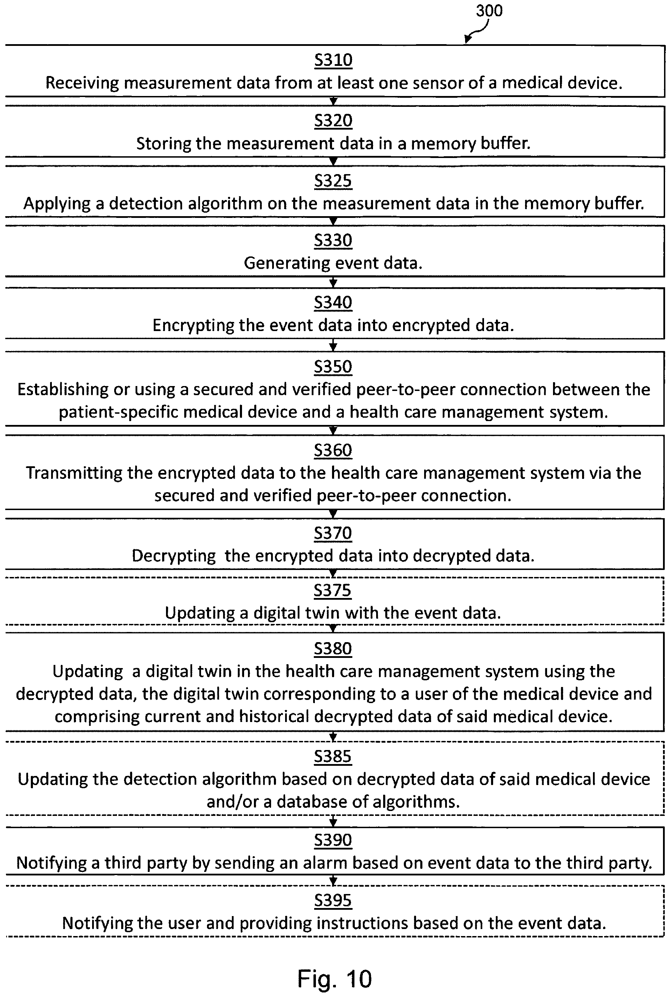

Fig. 10 shows a method 300 for bidirectional communication between a patient-specific medical device with at least one sensor and a health care management system (HCMS).

The method 300 comprises a number of steps, some of which are optional. The optional steps are marked in Fig. 10 with dashed boxes. Steps may be performed in a different order or simultaneously.

The first step is receiving S310 measurement data from at least one sensor of a medical device. This step S310 may be performed e.g. by a processing circuit or memory buffer of the medical device. This step S310 may further be performed by a separate device from the medical device, such as a personal server device.

The next step is storing S320 the measurement data in a memory buffer. This step S320 may be performed by the medical device or by a separate device, such as a personal server device.

Storing S320 may further comprise storing parameters of the medical device at a time of measuring.

Storing S320 may further comprise storing an absolute time stamp for each measurement or set of measurements corresponding to the measurement data in the memory buffer.

The next step is applying S325 a detection algorithm on the measurement data in the memory buffer. This step S325 may be performed by (a processing circuit of) the medical device or by a separate device, such as a personal server device.

The detection algorithm may be configured to detect a change in sensor data, a sudden presence or absence of data or pattern in the sensor data, and/or an event flag.

The detection algorithm may be further configured to determine that, based on the data being analyzed, a specific, future range or set of measurement data may be more likely to be relevant for an event; and store said specific, future range or set of measurement data as an event flag to be detected in the future.

When the detection algorithm detects an event, the next step is generating S330 event data. This step S330 may be performed by (a processing circuit of) the medical device or by a separate device, such as a personal server device.

Event data comprises data in the memory buffer related to the event detected by the detection algorithm and an event type based on the event detected by the detection algorithm.

Event data may further comprise an absolute time stamp of the event, position data of the user, duration and intensity of the event, and/or parameters of the medical device at the time of the event.

The next step is encrypting S340 the event data (and potentially data in the memory buffer) into encrypted data. This step S340 may be performed e.g. by a processing circuit on the event data and some or all of the data in the memory buffer.

The next step is establishing or using S350 a secured and verified peer-to-peer connection between the patient-specific medical device and the health care management system. This step S350 may be performed by the medical device, a network module (of the medical device or another device), and/or a connectivity server.

Establishing S350 a secured and verified peer-to-peer connection may comprise establishing a bidirectional system for verifying integrity of data being transmitted using the connection.

Establishing S350 a secured and verified peer-to-peer connection may comprise using a dedicated connectivity server to dynamically mediate the direct peer-to-peer connection by providing respective IP-addresses to the patient-specific medical device and the health care management system.

The next step is transmitting S360 the encrypted data to the health care management system via the secured and verified peer-to-peer connection. This step S360 may be performed by the medical device or a network module (of the medical device or another device).

A virtual channel may be assigned for each data or group of data being transmitted via the secured and verified peer-to-peer connection, wherein each virtual channel has a relative priority and data comprising event data has a highest priority.

The next step is decrypting S370 the encrypted data into decrypted data. This step S370 is performed by the HCMS.

The next step is updating S375 a digital twin with the event data. This step S375 is optional and performed by the HCMS.

The next step is updating S380 the digital twin in the health care management system (in real-time) using the decrypted data, the digital twin corresponding to a user of the medical device and comprising decrypted data of said medical device. This step S380 is performed by the HCMS.

Updating S380 the digital twin may comprise maintaining the same measurement data and parameters in the medical device and the digital twin.

The next step is updating S385 the detection algorithm based on decrypted data of said medical device and/or a database of algorithms. This step S385 is optional and performed by the HCMS.

Updating S385 the detection algorithm may be based on (previously collected) decrypted data of said medical device and/or a database of algorithms.

The next step is notifying S390 a third party by sending a notification/alarm based on event data to the third party. This step S390 is performed by the HCMS.

Notifying S390 a third party may comprise the health care management system selecting a third party to notify based on contact data of the user of the digital twin and sending a notification/alarm to the third party.

The third party notified S390 may be based on the (content of the) event data.

The next step is notifying S395 the user and providing instructions based on the event data. This step S395 is optional and performed by the HCMS.

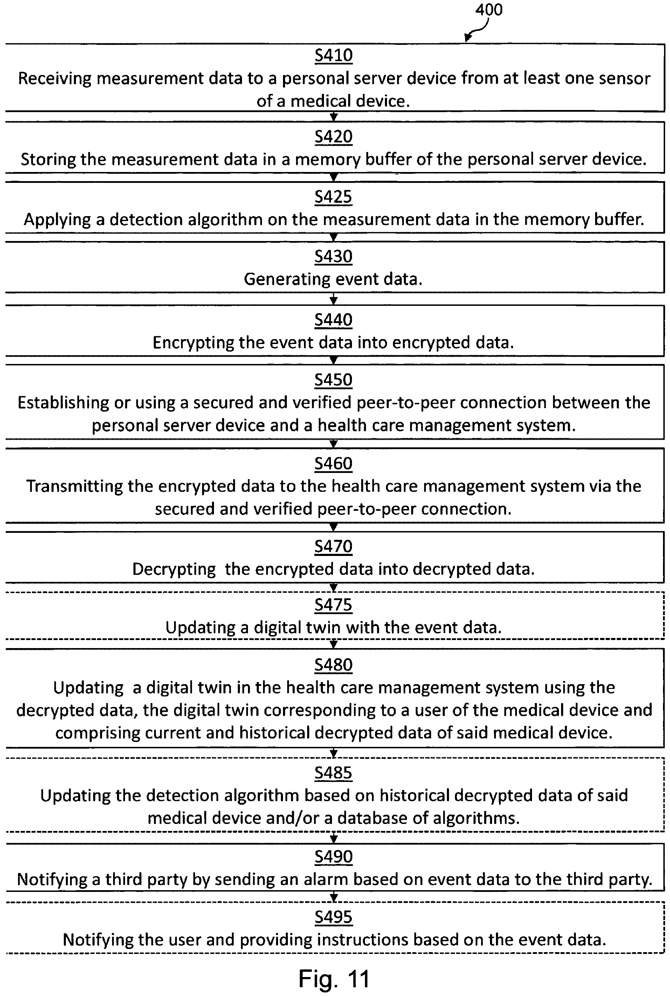

Fig. 11 shows a method 400 for bidirectional communication between a patient-specific medical device with at least one sensor and a health care management system (HCMS) via a personal server device.

The method 400 comprises a number of steps, some of which are optional. The optional steps are marked in Fig. 11 with dashed boxes. Steps may be performed in a different order or simultaneously.

The first step is receiving S410 measurement data to the personal server device from at least one sensor of the medical device. This step S410 may be performed e.g. by an antenna, a processing circuit, or a memory buffer of the personal server device.

The next step is storing S420 the measurement data in a memory buffer of the personal server device. This step S420 is performed by the personal server device.

Storing S420 may further comprise storing parameters of the medical device at a time of measuring.

Storing S420 may further comprise storing an absolute time stamp for each measurement or set of measurements corresponding to the measurement data in the memory buffer.

The next step is applying S425 a detection algorithm on the measurement data in the memory buffer. This step S425 may be performed by (a processing circuit of) the personal server device.

The detection algorithm may be configured to detect a change in sensor data, a sudden presence or absence of data or pattern in the sensor data, and/or an event flag.

The detection algorithm may be further configured to determine that, based on the data being analyzed, a specific, future range or set of measurement data may be more likely to be relevant for an event; and store said specific, future range or set of measurement data as an event flag to be detected in the future.

When the detection algorithm detects an event, the next step is generating S430 event data. This step S430 is performed by (a processing circuit of) the personal server device.

Event data comprises data in the memory buffer related to the event detected by the detection algorithm and an event type based on the event detected by the detection algorithm.

Event data may further comprise an absolute time stamp of the event, position data of the user, duration and intensity of the event, and/or parameters of the medical device at the time of the event.

The next step is encrypting S440 the event data (and potentially data in the memory buffer) into encrypted data. This step S440 may be performed e.g. by a processing circuit on the event data and some or all of the data in the memory buffer.

The next step is establishing or using S450 a secured and verified peer-to-peer connection between the personal server device and the health care management system. This step S450 may be performed by the personal server device and/or a connectivity server.

Establishing S450 a secured and verified peer-to-peer connection may comprise establishing a bidirectional system for verifying integrity of data being transmitted using the connection.

Establishing S450 a secured and verified peer-to-peer connection may comprise using a dedicated connectivity server to dynamically mediate the direct peer-to-peer connection by providing respective IP-addresses to the personal server device and the health care management system.

The next step is transmitting S460 the encrypted data to the health care management system via the secured and verified peer-to-peer connection. This step S460 is performed by the personal server device.

A virtual channel may be assigned for each data or group of data being transmitted via the secured and verified peer-to-peer connection, wherein each virtual channel has a relative priority and data comprising event data has a highest priority.

The next step is decrypting S470 the encrypted data into decrypted data. This step S470 is performed by the HCMS.

The next step is updating S475 a digital twin with the event data. This step S475 is optional and performed by the HCMS.

The next step is updating S480 the digital twin in the health care management system (in real-time) using the decrypted data, the digital twin corresponding to a user of the medical device and comprising decrypted data of said medical device. This step S480 is performed by the HCMS.

Updating S480 the digital twin may comprise maintaining the same measurement data and parameters in the medical device and the digital twin.

The next step is updating S485 the detection algorithm based on decrypted data of said medical device and/or a database of algorithms. This step S485 is optional and performed by the HCMS.

Updating S485 the detection algorithm may be based on (previously collected) decrypted data of said medical device and/or a database of algorithms.

The next step is notifying S490 a third party by sending a notification/alarm based on event data to the third party. This step S490 is performed by the HCMS.

Notifying S490 a third party may comprise the health care management system selecting a third party to notify based on contact data of the user of the digital twin and sending a notification/alarm to the third party.

The third party notified S490 may be based on the (content of the) event data.

The next step is notifying S495 the user and providing instructions based on the event data. This step S495 is optional and performed by the HCMS.

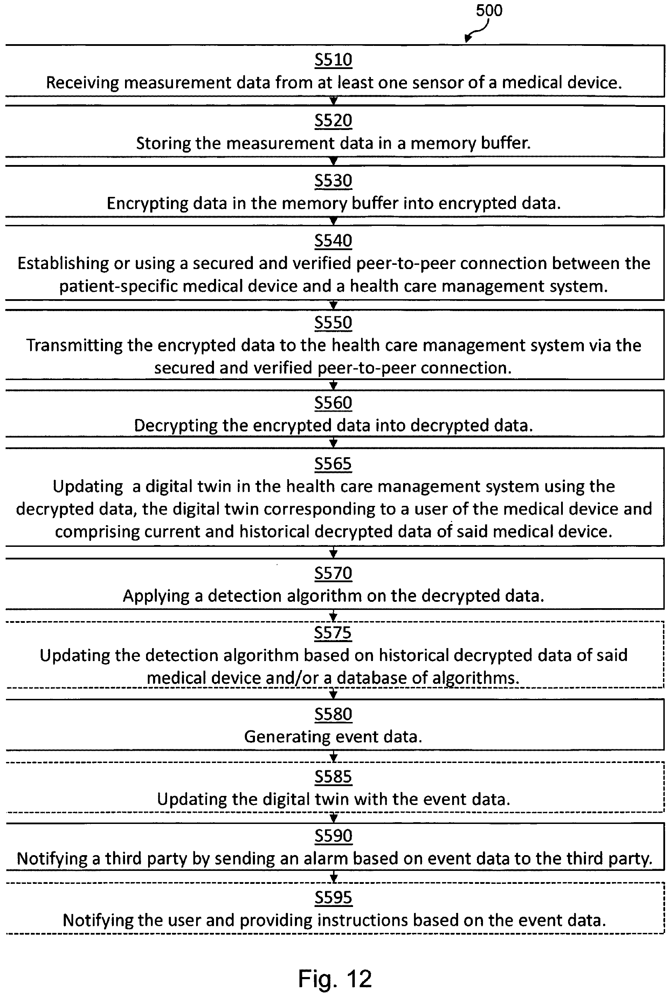

Fig. 12 shows a method 500 for bidirectional communication between a patient-specific medical device with at least one sensor and a health care management system (HCMS).

The method 500 comprises a number of steps, some of which are optional. The optional steps are marked in Fig. 12 with dashed boxes. Steps may be performed in a different order or simultaneously.

The first step is receiving S510 measurement data from at least one sensor of a medical device. This step S510 may be performed e.g. by a processing circuit or memory buffer of the medical device. This step S510 may further be performed by a separate device from the medical device, such as a personal server device.

The next step is storing S520 the measurement data in a memory buffer. This step S520 may be performed by the medical device or by a separate device, such as a personal server device.

Storing S520 may further comprise storing parameters of the medical device at a time of measuring.

Storing S520 may further comprise storing an absolute time stamp for each measurement or set of measurements corresponding to the measurement data in the memory buffer.

The next step is encrypting S530 data in the memory buffer into encrypted data. This step S530 may be performed e.g. by a processing circuit on some or all of the data in the memory buffer.

The next step is establishing or using S540 a secured and verified peer-to-peer connection between the patient-specific medical device and the health care management system. This step S540 may be performed by the medical device, a network module (of the medical device or another device), and/or a connectivity server.

Establishing S540 a secured and verified peer-to-peer connection may comprise establishing a bidirectional system for verifying integrity of data being transmitted using the connection.

Establishing S540 a secured and verified peer-to-peer connection may comprise using a dedicated connectivity server to dynamically mediate the direct peer-to-peer connection by providing respective IP-addresses to the patient-specific medical device and the health care management system.

The next step is transmitting S550 the encrypted data to the health care management system via the secured and verified peer-to-peer connection. This step S550 may be performed by the medical device or a network module (of the medical device or another device).

A virtual channel may be assigned for each data or group of data being transmitted via the secured and verified peer-to-peer connection, wherein each virtual channel has a relative priority.

The next step is decrypting S560 the encrypted data into decrypted data. This step S560 is performed by the HCMS.

The next step is updating S565 the digital twin in the health care management system (in real-time) using the decrypted data, the digital twin corresponding to a user of the medical device and comprising decrypted data of said medical device. This step S565 is performed by the HCMS.

Updating S565 the digital twin may comprise maintaining the same measurement data and parameters in the medical device and the digital twin.

The next step is applying S570 a detection algorithm on the decrypted data (in the digital twin). This step S570 is performed by the HCMS.

The detection algorithm may be configured to detect a change in sensor data, a sudden presence or absence of data or pattern in the sensor data, and/or an event flag.

The detection algorithm may be further configured to determine that, based on the data being analyzed, a specific, future range or set of decrypted data may be more likely to be relevant for an event; and store said specific, future range or set of decrypted data as an event flag to be detected in the future.

The next step is updating S575 the detection algorithm based on decrypted data of said medical device and/or a database of algorithms. This step S575 is optional and performed by the HCMS.

Updating S575 the detection algorithm may be based on (previously collected) decrypted data of said medical device and/or a database of algorithms.

When the detection algorithm detects an event, the next step is generating S580 event data. This step S580 is performed by the HCMS.

Event data comprises data in the digital twin related to the event detected by the detection algorithm and an event type based on the event detected by the detection algorithm.

Event data may further comprise an absolute time stamp of the event, position data of the user, duration and intensity of the event, and/or parameters of the medical device at the time of the event.

The next step is updating S585 the digital twin with the event data. This step S585 is optional and performed by the HCMS.

The next step is notifying S590 a third party by sending a notification/alarm based on event data to the third party. This step S590 is performed by the HCMS.

Notifying S590 a third party may comprise the health care management system selecting a third party to notify based on contact data of the user of the digital twin and sending a notification/alarm to the third party.

The third party notified S590 may be based on the (content of the) event data.

The next step is notifying S595 the user and providing instructions based on the event data. This step S595 is optional and performed by the HCMS.

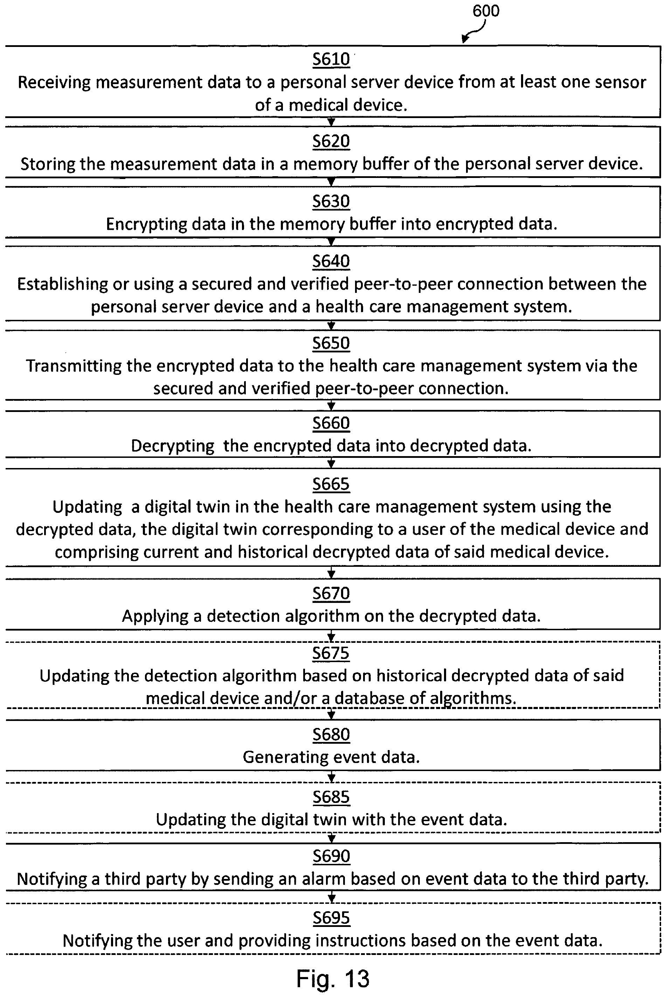

Fig. 13 shows a method 600 for bidirectional communication between a patient-specific medical device with at least one sensor and a health care management system (HCMS) via a personal server device.

The method 600 comprises a number of steps, some of which are optional. The optional steps are marked in Fig. 13 with dashed boxes. Steps may be performed in a different order or simultaneously.

The first step is receiving S610 measurement data to the personal server device from at least one sensor of the medical device. This step S610 may be performed e.g. by an antenna, a processing circuit, or a memory buffer of the personal server device.

The next step is storing S620 the measurement data in a memory buffer of the personal server device. This step S620 is performed by the personal server device.

Storing S620 may further comprise storing parameters of the medical device at a time of measuring.

Storing S620 may further comprise storing an absolute time stamp for each measurement or set of measurements corresponding to the measurement data in the memory buffer.

The next step is encrypting S630 data in the memory buffer into encrypted data. This step S630 may be performed e.g. by a processing circuit on some or all of the data in the memory buffer.

The next step is establishing or using S640 a secured and verified peer-to-peer connection between the personal server device and the health care management system. This step S640 may be performed by the personal server device and/or a connectivity server.

Establishing S640 a secured and verified peer-to-peer connection may comprise establishing a bidirectional system for verifying integrity of data being transmitted using the connection.

Establishing S640 a secured and verified peer-to-peer connection may comprise using a dedicated connectivity server to dynamically mediate the direct peer-to-peer connection by providing respective IP-addresses to the personal server device and the health care management system.

The next step is transmitting S650 the encrypted data to the health care management system via the secured and verified peer-to-peer connection. This step S650 is performed by the personal server device.

A virtual channel may be assigned for each data or group of data being transmitted via the secured and verified peer-to-peer connection, wherein each virtual channel has a relative priority.

The next step is decrypting S660 the encrypted data into decrypted data. This step S660 is performed by the HCMS.

The next step is updating S665 the digital twin in the health care management system (in real-time) using the decrypted data, the digital twin corresponding to a user of the medical device and comprising decrypted data of said medical device. This step S665 is performed by the HCMS.

Updating S665 the digital twin may comprise maintaining the same measurement data and parameters in the medical device and the digital twin.

The next step is applying S670 a detection algorithm on the decrypted data (in the digital twin). This step S670 is performed by the HCMS.

The detection algorithm may be configured to detect a change in sensor data, a sudden presence or absence of data or pattern in the sensor data, and/or an event flag.

The detection algorithm may be further configured to determine that, based on the data being analyzed, a specific, future range or set of decrypted data may be more likely to be relevant for an event; and store said specific, future range or set of decrypted data as an event flag to be detected in the future.

The next step is updating S675 the detection algorithm based on decrypted data of said medical device and/or a database of algorithms. This step S675 is optional and performed by the HCMS.

Updating S675 the detection algorithm may be based on (previously collected) decrypted data of said medical device and/or a database of algorithms.

When the detection algorithm detects an event, the next step is generating S680 event data. This step S680 is performed by the HCMS.

Event data comprises data in the digital twin related to the event detected by the detection algorithm and an event type based on the event detected by the detection algorithm.

Event data may further comprise an absolute time stamp of the event, position data of the user, duration and intensity of the event, and/or parameters of the medical device at the time of the event.

The next step is updating S685 the digital twin with the event data. This step S685 is optional and performed by the HCMS.

The next step is notifying S690 a third party by sending a notification/alarm based on event data to the third party. This step S690 is performed by the HCMS.

Notifying S690 a third party may comprise the health care management system selecting a third party to notify based on contact data of the user of the digital twin and sending a notification/alarm to the third party.

The third party notified S690 may be based on the (content of the) event data.

The next step is notifying S695 the user and providing instructions based on the event data. This step S695 is optional and performed by the HCMS.

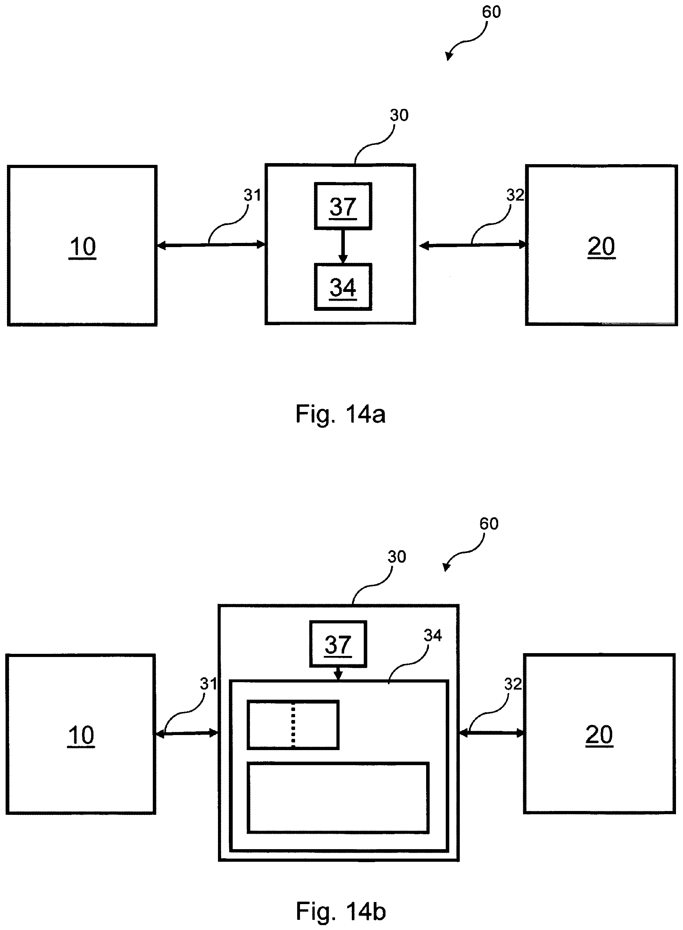

Fig. 14 shows a schematic view of a system 60 for managing limited temporary data storage in a portable personal server device 30. The system 60 comprises the portable personal server device (PS) 30, one or more patient-specific medical devices (MD) 10, and a health care management system (HCMS) 20.

The one or more MD 10 comprise at least one sensor and are configured to store measurement data received from said at least one sensor in a memory buffer and potentially to encrypt the data into encrypted data.

The HCMS 20 is configured to decrypt the encrypted data into decrypted data (if necessary) and to maintain a digital twin corresponding to a user of said one or more MD 10 comprising (decrypted) data of said one or more MD 10.

The digital twin is maintained by periodically updating the digital twin to comprise a copy of the measurement data and parameters as in said one or more MD 10.

The digital twin is a virtual copy of the MD, so any changes to the digital twin is mirrored in the MD 10 and vice versa. This not only lets the system 60 be simple and intuitive to operate as the MD 10 is controlled through remote via the HCMS 20, it also increases security as local control over the MD 10 may be blocked.

The digital twin is not a simulation of the MD 10, for all intents and purposes it is the MD 10. It's a digital interface to analyse and control the MD 10.

The MD 10 and the HCMS 20 may be similar to the ones described in relation to Figs. 1-5.

The PS 30 comprises a first wireless communication radio for using a local wireless connection 31 and a second wireless communication radio for using a secured and verified peer-to-peer connection 32. The local wireless connection 31 is used with said one or more MD 10 via the first wireless communication radio for transmitting data to and from the MD 10. The secured and verified peer-to-peer connection 32 is used with the HCMS 20 via the second wireless communication radio for transmitting data to and from the HCMS 20.

The secured and verified peer-to-peer connection 32 used with the HCMS 20 may be implemented using a dedicated connectivity server as described in relation to Fig. 2. This enables hole-punching through firewalls and for the HCMS 20 and PS 30 to communicate directly with each other with no intermediaries, including the connectivity server itself.

The dedicated connectivity server dynamically mediates the direct peer-to-peer connection 32 by providing respective IP-addresses to the PS 30 and the HCMS 20. In this way, the portable PS does not need a fixed IP address and can use any other unique ID instead such as a serial number or a MAC address. This enables the system 60 to be more flexible and reliable, as well as more secure.

The PS 30 further comprises a primary memory buffer 34 and a memory processing circuit 37.

The PS 30 is configured to receive data from the MD 10 using the local wireless connection 31, the data comprising a time stamp corresponding to a time of measurement of the corresponding measurement data.

The PS 30 may be configured to store data in the primary memory buffer 34 as transmission-ready packages.

The memory processing circuit 37 is configured to assign a first priority to the data proportional to and increasing with an age of the time stamp; and a second priority to the data inversely proportional to and decreasing with the age of the time stamp. These priorities and the data are stored in the primary memory buffer 34 while awaiting transmission.

The PS 30 is configured to transmit the data to the HCMS 20 using the secured and verified peer-to-peer connection 32 in order according to the second priority. Accordingly, newer data are sent first, as this may be more important.

Once data are transmitted, they are deleted after receiving confirmation from the HCMS 20 that the data have been received through the secured and verified peer-to-peer connection 32.

In case the secured and verified peer-to-peer connection 32 is unreliable, which may be a consequence of the PS being portable as a user may be moving through a tunnel or into a basement, the data may not be able to be transmitted to the HCMS for long-term storage.

When this happens, the primary memory buffer 34 may get full while still receiving data from the MD 10. As this new data may be even more important than the data currently being stored in the primary memory buffer 34, the memory processing circuit 37 is configured to delete non-transmitted data in order according to the first priority to make space when storage capacity in the primary memory buffer 34 reaches a predetermined storage limit. By deleting data in order according to the first priority, the oldest and thereby potentially least relevant data are deleted first.

The first priority may be determined according to a first algorithm and the second priority may be determined according to a second algorithm with a different proportionality to the age of the time stamp.

A different proportionality may be used to assign the first and second priorities depending on a type of the measurement data.

For example, the first algorithm may be an exponential curve for heart rate and a linear curve for EEG measurements, whereas the second algorithm is a linearly declining curve for both heart rates and EEG measurements, but they start at different values and/or have a different rate of decline.

It may be relevant to have a different proportionality or algorithm for the first and second priorities because it may be especially important to transmit new data to e.g. ensure a digital twin of the HCMS 20 is up to date, whereas the type or content of the data may be more relevant when considering which data to delete first. Age is a factor here as well, but it may for example decay slower.

The data transmitted to the HCMS 20 by the PS 30 is transmitted purposefully out of order to accommodate an unreliable bandwidth of the secured and verified peer-to-peer connection 32 and the principle that different data have different importance. As all the data have time stamps, the HCMS 20 will be able to restore the data to a correct timeline and put them in order again.

The HCMS 20 will notice if data are missing as there will be empty slots in a reconstructed timeline where the HCMS 20 would expect there to be data. The HCMS 20 may also notice if there is/was a reduction or break in receiving data from the PS 30.

The HCMS 20 may automatically assume the missing data to be lost due to a memory buffer limit and/or the HCMS 20 may ask the PS 30 whether data has been deleted.

The PS 30 may keep track of whether it has deleted data and how much data have been deleted. This may be simply implemented using a counter to save on memory space, but a more involved register may also be used.

If a counter is used, this counter may be transmitted to the HCMS 20 periodically and/or after an interruption of communications to inform the HCMS 20 to expect that data will be missing and how much.

The memory processing circuit 37 may further be configured to assign a third priority to the data depending on a type of the data. The third priority may e.g. reflect certain flags or metadata of a package such as one that would indicate an alarm (which has an inherently high priority) or it may reflect a difference between a single data point and a data set (or data belonging to a larger data set). The third priority may also reflect which sensor the data came from, as certain sensors may be considered more important than others in a study or for a patient's health.

The third priority is not proportional to an age of the data, it is inherent to the data itself and its metadata.

The third priority may impact either or both of the implementations of priority, i.e. it may impact the transmission order of data and/or the deletion order of data.

The third priority may combined with the first and/or second priority as a factor, an addition, or a completely separate reference point. In some embodiments, the third priority is checked primarily and the first and/or second priority is checked secondarily.

The third priority may be high enough or implemented in a way to completely prevent certain data from ever being deleted and ensure it is always transmitted first. This may allow life-threatening data to navigate the primary memory buffer 34 as efficiently as possible.

Fig. 14b shows a practical implementation of the concepts in Fig. 14a with limited hardware.

If the proportionality of the first and second priorities are the same for all data, the primary memory buffer 34 implements a last in first out (LIFO) stack. However, such a LIFO stack may be difficult to implement with write-limited memory types such as flash, which is better suited for first in first out (FIFO) stacks.

Fig. 14b shows an implementation of a sort of LIFO stack using two sequential FIFO stacks. The first FIFO stack is one memory shown at the top of the primary memory buffer 34, and the second FIFO stack is another memory shown at the bottom of the primary memory buffer 34.

In this example the first FIFO is a PSRAM and the second FIFO is a flash memory, but other memory structures and combinations are possible.

The first FIFO stack has a high second priority, which means that transmissions to the long-term storage will prioritize packages from the first FIFO stack over the second FIFO stack. Preferably this transmission will be fast enough during normal operations that the stack is not used much so that the difference whether this is LIFO or FIFO is negligible.

The main purpose of this first FIFO stack is to store data as it is being transmitted before it can be deleted after verification that the long-term storage has received it.

If transmissions are interrupted for any reason the first FIFO stack will become full and data in the first FIFO stack is continually moved to the second FIFO stack. The second FIFO stack has a high first priority, which means that if both FIFO stacks fill up the data in the second FIFO stack are deleted first.

Together, the first and second FIFO stacks implement a primary memory buffer 34 as in Fig. 14. This movement and priority of data/memories is be controlled by a memory processing circuit 37.

Adding a third priority may be done by partitioning the first FIFO, as indicated by the dashed line in the first FIFO. This partitioning may be even or uneven, i.e. each side may be the same or a different size after the partitioning. In Fig. 14b this partitioning is even.

In this case data with a high third priority will be stored in the first half of the first FIFO and data with low third priority will be stored in the second half of the first FIFO, effectively creating two FIFO stacks.

The data in the first half will be sent first and the data in the second half will be moved to the second FIFO stack if space is needed.

Accordingly, the PS 30 takes into account both the first and third priorities when deleting data and both the second and third priorities when transmitting data.

Partitioning the first FIFO in such a way may be especially beneficial in that two shorter FIFO stacks are created, and FIFO stacks are more efficient and more similar to LIFO stacks the smaller they are.

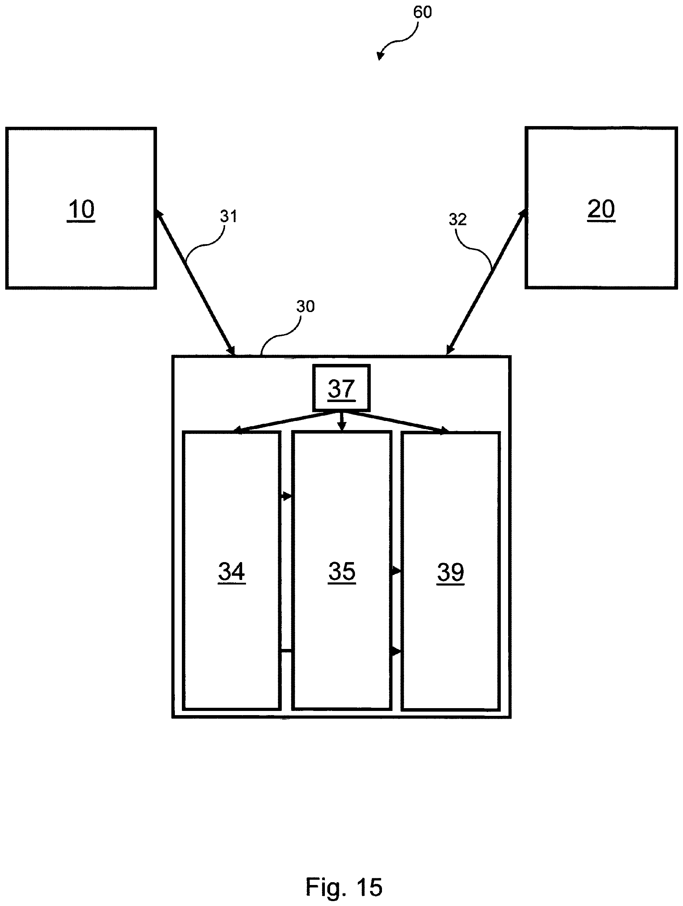

Fig. 15 shows a schematic view of a system 60 for managing limited temporary data storage in a PS 30. Compared to the system 60 in Figs. 14a-b, Fig. 15 also comprises a secondary memory buffer 35 and a tertiary memory buffer 39.

The secondary memory buffer 35 has a lower read/write speed and/or a higher energy cost than the primary memory buffer 34. For example, the primary memory buffer 24 may be an SRAM memory, the secondary memory buffer 35 may be a PSRAM memory, and the tertiary memory buffer 39 may be a flash memory.

Comparatively, the SRAM has fast read/write speed, has low energy cost, is volatile and has a small memory capacity. The PSRAM has medium read/write speed, has medium energy cost, is volatile and has a medium memory capacity. The flash has medium read/write speed, has high energy cost, is non-volatile and has a large memory capacity.

The system 60 in Fig. 15 has a single secondary memory buffer 35 and a single tertiary memory buffer 39, but any number of secondary memory buffers 35 and tertiary memory buffers 39 are possible. A single memory buffer may be both a secondary memory buffer 35 and a tertiary memory buffer 39.

Two partitions of the same memory may be a primary memory buffer 34 in one partition and a secondary memory buffer 35 and/or a tertiary memory buffer 39 in another partition.

The memory processing circuit 37 is configured to move data from the primary memory buffer 34 to the secondary memory buffer 35 in order according to the first and/or third priority when storage capacity in the primary memory buffer 34 reaches the predetermined storage limit.

This is done instead of deleting data (it may also be considered that moving data implicitly involves deleting data as part of the move, the important distinction is that no data in the primary memory buffer 34 is lost) in the primary memory buffer 34 when storage capacity in the primary memory buffer 34 reaches the predetermined storage limit. Instead, the memory processing circuit 37 is configured to delete data in the secondary memory buffer 35 in order according to the first and/or the third priority when storage capacity in the secondary memory buffer 35 reaches a predetermined storage limit.

The predetermined storage limit of the secondary memory buffer 35 may be the same as or different to the predetermined storage limit of the primary memory buffer 34.

Accordingly, the data flow is maintained in the primary memory buffer 34 and only data in the secondary memory buffer 35 is deleted. The data in the secondary memory buffer 35 is less important than the data in the primary memory buffer 34 because of priority, and also when deleting data in the secondary memory buffer 35 priority is used to ensure that only the least important data is permanently deleted.

In cases with more than one unit implementing the secondary memory buffer 35, each unit may be assigned a rank according to its read/write speed and/or energy cost and data is moved from unit to unit following rank in the same manner as moving data from the primary memory buffer 34 to the secondary memory buffer 35 such that read/write speed keeps decreasing and/or energy cost keeps increasing, balancing these conditions against each other when necessary.

Also when deleting data from the secondary memory buffer 35, only data in the lowest ranked unit may be deleted and the rest is moved down. However, in some cases data in units that are not the lowest ranked unit may instead or also be deleted to avoid processing a lot of data in the lowest ranked, least efficient unit.

The rank of different units may further be affected by the possibility to rewrite the same memory space, and memory types that have rewrite limitations may only be used to store overflow data and not be the units that are cleared to make room for more data. These units may have the lowest rank, but may not be considered for when data is to be deleted.

Accordingly, when deleting data from the secondary memory buffer 35, only data in the lowest ranked unit that is at least partially or fully rewriteable may be deleted. This may only be done when all the units of the secondary memory buffer 35 are full (or reach a predetermined storage limit), including the ones with limited rewriteability.

The memory processing circuit 37 is configured to move data from the primary memory buffer 34 to the tertiary memory buffer 39 in order according to the first and/or the third priority when a battery level of the PS 30 reaches a predetermined charge.

It may be important to preserve data in the memory buffer(s) 34. 35 if the PS runs out of battery.

If the primary memory buffer 34 is volatile, a backup system may be to move any non-transmitted data to the non-volatile tertiary memory buffer 39 before the battery runs out.

When battery power is restored, the data may be moved back to the primary memory buffer 34 and/or be transmitted directly from the tertiary memory buffer 39 if bandwidth allows.

If a secondary memory buffer 35 is present, depending on the volatility of that secondary memory buffer 35, the memory processing circuit 37 may be configured to move data from the secondary memory buffer 35 to the tertiary memory buffer 39 in a similar manner as when moving the data from the primary memory buffer 34.

Should the tertiary memory buffer 39 run out of memory space, the priority means that at least the most important data is present in the non-volatile tertiary memory buffer 39 and able to be transmitted once battery power is restored.

The memory processing circuit 37 may be more or less intelligent depending on available processing power. The memory processing circuit 37 may apply a buffer storage algorithm that takes into account data type, data metadata, data age, battery status of the PS 30, and memory rank.

For example, a patient may be wearing an EEG sensor device and a heartrate monitor. The memory processing circuit 37 may take into account that the EEG sensor device produces a large volume of data and that most of the time the data does not contain anything relevant for the current study, such as a spike or a seizure. The memory processing circuit 37 may assign such "empty" data a high priority to be deleted first if memory space is needed, either through a specific algorithm determining the first priority or by using a combination of the first and third priority. The data is also less relevant the older it is, so the first priority increases with age. However, the memory processing circuit 37 may assign all the EEG data a high priority to be transmitted compared to the heartrate data, even if it is older, because the EEG data is the main focus of the study and the heartrate data is considered secondary. This may be done either through different algorithms determining the second priority of the EEG data and the heartrate data or by using a combination of the second and third priority. Still, the newest EEG data should be transmitted first so its second priority decreases with age. However, if the EEG data comprises a seizure, which may be noted in the metadata, this data should never be deleted and always transmitted first, circumventing all other priorities. This may be done either through different algorithms determining the first and second priority depending on the metadata or by using the third priority.

That is to say, the shorthand of first, second, and third priorities may belie a complicated evaluation of several different and competing factors to ensure that the PS 30 uses its memory in an optimized way.

The memory processing circuit 37 may use artificial intelligence and/or neural networks to assign different priorities (i.e. first, second and/or third priorities) to data. The memory processing circuit 37 may be pre-trained and/or train as it is being used on live data. The memory processing circuit 37 may become personalized to a patient using the PS 30 to better anticipate what data will be considered most important by healthcare professionals.

The memory processing circuit 37 may be pre-programmed with several different buffer algorithms and strategies for optimizing the buffer and it may apply one by default based on the received measurement data and/or one is selected by the patient or a healthcare professional.

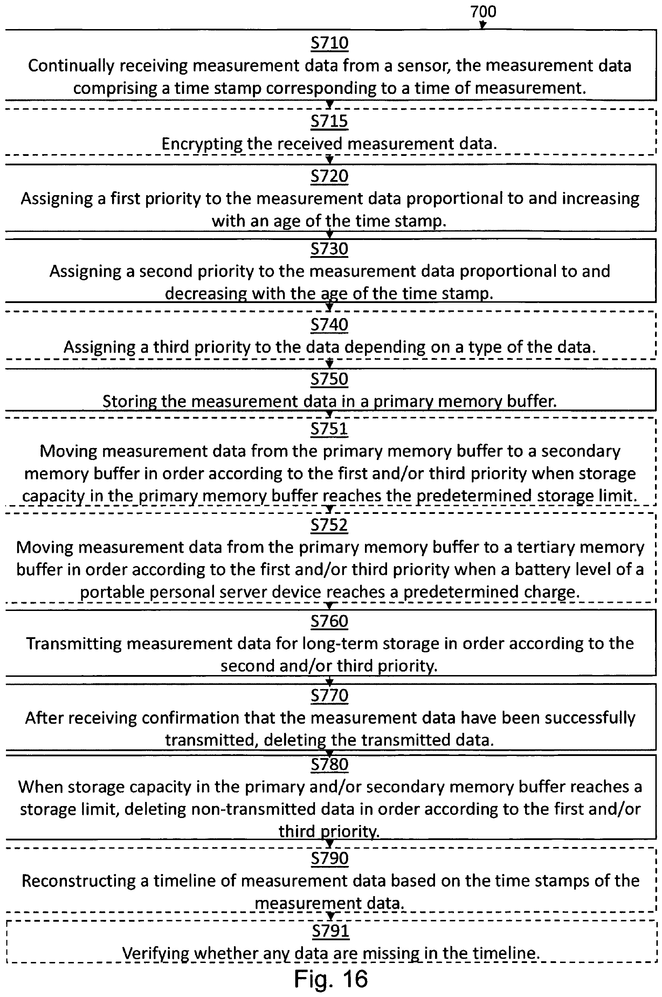

Fig. 16 shows a flowchart of a method 700 for managing limited temporary data storage in a portable device.

The method 700 comprises a number of steps, some of which are optional. The optional steps are marked in Fig. 16 with dashed boxes. Steps may be performed in a different order or simultaneously.

The first step is continually receiving S710 measurement data from at least one sensor, the measurement data comprising a time stamp corresponding to a time of measurement.

This step may be performed by a PS as previously described.

The time stamp is created when measurement occurs and is recorded by the MD. An internal clock of the MD may be synched with the PS such that several MDs connected to the same PS have synched time stamps.

The next step is encrypting S715 the received measurement data. This step may be performed by a PS as previously described.

This step is optional, and may not be necessary if the received measurement data is already encrypted. This step may still be performed is the received measurement data is already encrypted if a different encryption is wanted, such as one adapted for secure transmission or for receipt by the long-term storage.

The next step is assigning S720 a first priority to the measurement data proportional to and increasing with an age of the time stamp. This step may be performed by a memory processing circuit of a PS as previously described.

This may comprise applying an algorithm to the measurement data.

The next step is assigning S730 a second priority to the measurement data proportional to and decreasing with the age of the time stamp. This step may be performed by the memory processing circuit of a PS as previously described.

This may comprise applying an algorithm to the measurement data, which may be different to the algorithm applied to assign S720 the first priority.

The next step is assigning S740 a third priority to the measurement data depending on a type of the data. This step may be performed by the memory processing circuit of a PS as previously described.

The type of data may be determined by a source of the data and/or a format or metadata of the data.

The next step is storing S750 the measurement data in a primary memory buffer. This step may be performed by the memory processing circuit of a PS as previously described.

The memory processing circuit may place the data within the memory buffer with care to increase the lifetime of the memory and improve efficiency.

The next step is moving S751 measurement data from the primary memory buffer to a secondary memory buffer in order according to the first and/or third priority when storage capacity in the primary memory buffer reaches the predetermined storage limit.

This step is optional and may be performed by the memory processing circuit of a PS as previously described.

The secondary memory buffer may have a lower read/write speed and/or a higher energy cost than the primary memory buffer.

By these priorities, the least important data is moved first so that the primary memory buffer always comprises the most important data.

Since the primary memory buffer may be considered of a higher quality (i.e. higher read/write speed and/or a lower energy cost) than the secondary memory buffer, the primary memory buffer should be used as much as possible. By this arrangement, less important data that may be stored for a longer period of time is moved to make room in the better memory buffer for more important data that will be processed first.

There may be several secondary memory buffers with different levels of quality. In such cases, the step of moving S751 measurement data from the primary memory buffer to a secondary memory buffer may be repeated to move data from a higher-quality secondary memory buffer to a lower-quality secondary memory buffer.

The next step is moving S752 measurement data from the primary memory buffer to a tertiary memory buffer in order according to the first and/or third priority when a battery level of the PS reaches a predetermined charge.

This step is optional and may be performed by the memory processing circuit of a PS as previously described.

The tertiary memory buffer may be less volatile than the primary memory buffer.

It may be important to preserve data in the memory buffer(s) if the PS runs out of battery. If the primary memory buffer is volatile a backup system may be to move any non-transmitted data to a non-volatile tertiary memory buffer before the battery runs out.

When battery power is restored, the data may be moved back to the primary memory buffer and/or be transmitted directly from the tertiary memory buffer.

If a secondary memory buffer is present, depending on the volatility of that secondary memory buffer, the step of moving S752 measurement data from the primary memory buffer to a tertiary memory buffer may be repeated for moving measurement data from the secondary memory buffer to a tertiary memory buffer mutatis mutandis.

The next step is transmitting S760 measurement data for long-term storage in order according to the second and/or third priority.

This step may be performed by the memory processing circuit of a PS as previously described.

By these priorities, the most important data is transmitted first in case connection is lost again.

The next step is after deleting S770 the transmitted data after receiving confirmation that the measurement data have been successfully transmitted.

This step may be performed by the memory processing circuit of a PS as previously described.

Since this data is now in long-term storage, the memory buffer(s) may be cleared to make room.

The next step is deleting S780 non-transmitted data in order according to the first and/or third priority when storage capacity in the primary and/or secondary memory buffer(s) reaches a storage limit.

This step may be performed by the memory processing circuit of a PS as previously described.

If connection to long-term storage is interrupted, the memory buffer(s) will fill up as the PS continues to receive new data. Accordingly, old data needs to be deleted so that new data may be received.

The non-transmitted data in the memory buffer(s) may be important medical data so it should be deleted with care and consideration. This is accomplished by using the first and/or third priority such that old and unimportant data are deleted first.

When a secondary memory buffer is used, data may only be deleted in the secondary memory buffer and only when the secondary memory buffer fills up. Data in the primary memory buffer is then only moved (and not deleted) to the secondary memory buffer when the primary memory buffer fills up. This way, data flow is maintained in the primary memory buffer so that transmission may be resumed efficiently as soon as possible after connection with long-term storage is reestablished.

The next step is reconstructing S790 a timeline of measurement data based on the time stamps of the measurement data.

This step is optional and may be performed by the HCMS as previously described.

This may comprise using the time stamps to populate a (historical) database in chronological order.

The next step is verifying S791 whether any data are missing in the timeline.

This step is optional and may be performed by the HCMS as previously described.

This may comprise checking the sampling frequency of the received data and looking for interruptions in an expected dataset.

The preceding description has been a non-exhaustive disclosure of different exemplifying embodiments of the present inventive concept. This is not to be misconstrued as limiting the scope of the protection sought for the inventive concept, which is defined by the appended claims.

## Claims

1. A portable personal server device (30) for facilitating a secured and verified peer-to-peer bidirectional data synchronization between one or more patient-specific medical devices (10) with at least one sensor (12) and a health care management system (20);
wherein the health care management system (20) is configured to maintain a digital twin (22) corresponding to said one or more patient-specific medical devices (10); and
wherein the personal server device (30) comprises:
a first wireless communication radio for using a local wireless connection (31);
a second wireless communication radio for using a secured and verified peer-to-peer connection (32);
a primary memory buffer (34); and
a memory processing circuit (37);
wherein the personal server device (30) is configured to:
use the local wireless connection (31) with said one or more patient-specific medical devices (10) via the first wireless communication radio for transmitting measurement data to and from said one or more patient-specific medical devices (10);
use the secured and verified peer-to-peer connection (32) with the health care management system (20) via the second wireless communication radio for transmitting measurement data to, and receiving parameter data comprising a time stamp from, the health care management system (20) to facilitate synchronization with the digital twin (22); and
receive measurement data from at least one sensor (12), the measurement data comprising a time stamp corresponding to a time of measurement;
wherein the memory processing circuit (37) is configured to:
assign a first priority to the measurement data proportional to and increasing with an age of its time stamp;
assign a second priority to the measurement data proportional to and decreasing with the age of its time stamp;
assign a fourth priority to the parameter data proportional to and increasing with an age of its time stamp;
assign a fifth priority to the parameter data proportional to and decreasing with an age of its time stamp; and
store the measurement data and parameter data in the primary memory buffer (34);
wherein the personal server device (30) is further configured to transmit the measurement data to the health care management system (20) in order according to the second priority; and
the personal server device (30) is further configured to transmit the parameter data to said one or more patient-specific medical devices (10) in order according to the fifth priority; and
wherein the memory processing circuit (37) is further configured to:
delete the transmitted measurement data after receiving confirmation that the measurement data have been successfully transmitted;
delete the transmitted configuration data after receiving confirmation that the parameter data have been successfully transmitted; and
delete non-transmitted data in order according to the first priority and/or the fourth priority when storage capacity in the primary memory buffer (34) reaches a predetermined limit.

2. The device according to claim 1, wherein:
the second wireless communication radio is used to create a peer-to-peer connection (32) between the personal server device (30) and the health care management system (20) such that they may communicate data directly with each other via the peer-to-peer connection (32) and through firewalls;
a dedicated connectivity server (40) dynamically mediates the direct peer-to-peer connection (32) by providing respective IP-addresses to the personal server device (30) and the health care management system (20); and
the data is not stored by the connectivity server (40) after the data transmission via the peer-to-peer connection (32).

3. The device according to claim 1 or 2, further configured to decrypt encrypted data received by said one or more patient-specific medical devices (10) and re-encrypt the data before transmitting the re-encrypted data to the health care management system (20).

4. The device according to claim 3, further configured to extract features from the decrypted data of said one or more patient-specific medical devices (10), to encrypt the extracted features, and to transmit the encrypted extracted features to the health care management system (20).

5. The device according to any one of the preceding claims, wherein the first priority is determined according to a first algorithm and the second priority is determined according to a second algorithm with a different proportionality to the age of the time stamp.

6. The device according to any one of the preceding claims, wherein a different proportionality is used to assign the first and second priorities depending on a type of the measurement data.

7. The device according to any one of the preceding claims, wherein the memory processing circuit (37) is further configured to assign a third priority to the measurement data depending on a type of the measurement data;
the personal server device (30) is configured to transmit the measurement data to the health care management system (20) in order according to the second and/or the third priority; and
the memory processing circuit (37) is configured to delete non-transmitted data in order according to the first and/or the third priority when storage capacity in the primary memory buffer (34) reaches the predetermined storage limit.

8. The device according to any one of the preceding claims, further comprising at least one secondary memory buffer (35) with a lower read/write speed and/or a higher energy cost than the primary memory buffer (34);
wherein the memory processing circuit (37) is configured to:
move measurement data from the primary memory buffer (34) to the secondary memory buffer (35) in order according to the first and/or third priority when storage capacity in the primary memory buffer (34) reaches the predetermined storage limit; and
delete data in the secondary memory buffer (35) in order according to the first and/or the third priority when storage capacity in the secondary memory buffer (35) reaches a predetermined secondary storage limit.

9. The device according to any one of the preceding claims, further comprising at least one tertiary memory buffer (39) less volatile than the primary memory buffer (34);
wherein the memory processing circuit (37) is configured to move measurement data from the primary memory buffer (34) to the tertiary memory buffer (39) in order according to the first and/or the third priority when a battery level of the portable personal server device (30) reaches a predetermined charge.

10. A method (700) for managing limited temporary data storage in a portable device, the method (700) comprising steps of:
continually receiving (S710) measurement data from at least one sensor of a medical device, the measurement data comprising a time stamp corresponding to a time of measurement;
periodically receiving (S711) parameter data from a health care management system, the parameter data comprising a time stamp;
assigning (S720) a first priority to the measurement data proportional to and increasing with an age of its time stamp;
assigning (S721) a fourth priority to the parameter data proportional to and increasing with an age of its time stamp;
assigning (S730) a second priority to the measurement data proportional to and decreasing with the age of its time stamp;
assigning (S731) a fifth priority to the parameter data proportional to and decreasing with an age of its time stamp;
storing (S750) the measurement data and parameter data in a primary memory buffer;
transmitting (S760) measurement data for long-term storage in order according to the second priority;
transmitting (S761) parameter data to the medical device in order according to the fifth priority;
after receiving confirmation that the measurement data have been successfully transmitted, deleting (S770) the transmitted measurement data;
after receiving confirmation that the parameter data have been successfully transmitted, deleting (S771) the transmitted parameter data; and
when storage capacity in the primary memory buffer reaches a predetermined storage limit, deleting (S780) non-transmitted data in order according to the first priority and/or the fourth priority.

11. The method according to claim 10, wherein the first priority is determined according to a first algorithm and the second priority is determined according to a second algorithm with a different proportionality to the age of the time stamp.

12. The method according to claim 10 or 11, wherein a different proportionality is used to assign the first and second priorities depending on a type of the measurement data.

13. The method according to any one of claims 10-12, further comprising steps of:
assigning (S740) a third priority to the measurement data depending on a type of the measurement data; wherein
transmitting (S760) measurement data for long-term storage is additionally or alternatively in order according to the third priority; and
deleting (S780) non-transmitted data is additionally or alternatively in order according to the third priority.

14. The method according to any one of claims 10-13, wherein the measurement data are received (S710), stored (S750), and transmitted (S760) in an encrypted state.

15. The method according to any one of claims 10-13, further comprising a step of encrypting (S715) the received (S710) measurement data; wherein the measurement data are stored (S750) and transmitted (S760) in an encrypted state.

16. The method according to any one of claims 10-15, further comprising steps of:
moving (S751) measurement data from the primary memory buffer to a secondary memory buffer in order according to the first and/or third priority when storage capacity in the primary memory buffer reaches the predetermined storage limit; and wherein
deleting (S780) data further comprises deleting data in the secondary memory buffer in order according to the first and/or the third priority when storage capacity in the secondary memory buffer reaches a predetermined secondary storage limit.

17. The method according to any one of claims 10-16, further comprising a step of moving (S752) measurement data from the primary memory buffer to a tertiary memory buffer in order according to the first and/or the third priority when a battery level of the portable personal server device reaches a predetermined charge.

18. The method according to any one of claims 10-17, further comprising a steps of:
reconstructing (S790) a timeline of measurement data based on the time stamps of the measurement data; and
verifying (S792) whether any data are missing in the timeline.

19. A system (60) for managing limited temporary data storage in a portable personal server device (30), the system (60) comprising:
one or more patient-specific medical devices (10) comprising at least one sensor (12), each medical device (10) being configured to store measurement data received from said at least one sensor (12) in a memory buffer (14) and to encrypt the data into encrypted data;
a health care management system (20) configured to decrypt the encrypted data into decrypted data and to maintain a digital twin (22) corresponding to a user (1) of said one or more patient-specific medical devices (10) comprising decrypted data of said one or more patient-specific medical devices (10); and
a portable personal server device (30) comprising:
a first wireless communication radio for using a local wireless connection (31);
a second wireless communication radio for using a secured and verified peer-to-peer connection (32); and
a primary memory buffer (34);
the personal server device (30) being configured to:
use a local wireless connection (31) with said one or more patient-specific medical devices (10) via the first wireless communication radio for transmitting encrypted data to and from said one or more patient-specific medical devices (10);
use a secured and verified peer-to-peer connection (32) with the health care management system (20) via the second wireless communication radio for transmitting encrypted data to, and receiving parameter data comprising a time stamp from, the health care management system (20) to facilitate synchronization with the digital twin (22);
receive measurement data from at least one sensor (12) using the local wireless connection (31), the measurement data comprising a time stamp corresponding to a time of measurement of the corresponding measurement data;
assig a first priority to the measurement data proportional to and increasing with an age of its time stamp;
assig a second priority to the measurement data proportional to and decreasing with the age of its time stamp;
assign a fourth priority to the parameter data proportional to and increasing with an age of its time stamp;
assign a fifth priority to the parameter data proportional to and decreasing with an age of its time stamp;
store the measurement data and parameter data in the primary memory buffer (34);
transmit the measurement data to the health care management system (20) using the secured and verified peer-to-peer connection (32) in order according to the second priority;
transmit the parameter data to said one or more patient-specific medical devices (10) in order according to the fifth priority;
delete the transmitted data after receiving confirmation that the measurement data has been successfully transmitted;
delete the transmitted configuration data after receiving confirmation that the parameter data have been successfully transmitted; and
delete non-transmitted data in order according to the first priority and/or the fourth priority when storage capacity in the primary memory buffer (34) reaches a predetermined storage limit.

20. The system according to claim 19, wherein the digital twin (22) is maintained by periodically updating the digital twin (22) to comprise a copy of the measurement data and parameters as in said one or more patient-specific medical device (10).

21. The system according to claim 19 or 20, further comprising a dedicated connectivity server (40) configured to dynamically mediate the peer-to-peer connection (32) by providing respective IP-addresses to the personal server device (30) and the health care management system (20); and to not store data after the data transmission via the peer-to-peer connection (32).

22. The system according to any one of claims 19-21, wherein the health care management system (20) is configured to reconstruct a timeline of decrypted data based on the time stamps of the received encrypted data and verify whether any data are missing in the timeline.

23. The system according to any one of claims 19-22, wherein the personal server device (30) is configured according to any one of claims 1-9.
